# EUROPÄISCHE PATENTSCHRIFT

(11) **EP 1 172 349 B1**
(45) Veröffentlichungstag und Bekanntmachung des Hinweises auf die Patenterteilung: **20.04.2005**
(21) Anmeldenummer: 01114109.0
(22) Anmeldetag: 09.06.2001
(51) Int. Cl.: C07C 45/50, C07C 45/82

(54) **Mehrstufiges Verfahren zur Herstellung von Oxo-Aldehyden und/oder Alkoholen**
Multi-step process for the preparation of oxo-aldehydes and/or alcohols
Procédé à plusieurs étapes pour la préparation d'oxo-aldéhydes et/ou d'alcools

(30) Priorität: 14.07.2000 DE 10034360
(43) Veröffentlichungstag der Anmeldung: 16.01.2002
(73) Patentinhaber: Oxeno Olefinchemie GmbH, 45764 Marl (DE)
(72) Erfinder: Kaizik, Alfred, Dr., 45772 Marl (DE); Scholz, Bernhard, Dr., 45768 Marl (DE); Röttger, Dirk, Dr., 45657 Recklinghausen (DE); Wiese, Klaus-Diether, Dr., 45721 Haltern (DE); Büschken, Wilfried, Dr., 45721 Haltern (DE); Hess, Dieter, Dr., 45770 Marl (DE); Nierlich, Franz, Dr., 45768 Marl (DE); Protzmann, Guido, Dr., 64673 Zwingenberg (DE)

(56) Entgegenhaltungen:
- EP-A- 0 111 257
- EP-A- 0 805 138
- EP-A- 0 987 243
- EP-A- 1 057 803

## Beschreibung

Die vorliegende Erfindung betrifft ein Verfahren zur Herstellung von Aldehyden mit 7 bis 25 Kohlenstoffatomen durch mehrstufige Kobalt- oder Rhodiumkatalysierte Hydroformylierung der entsprechenden Olefine.

### Stand der Technik

Höhere Aldehyde, insbesondere solche mit 7 bis 25 Kohlenstoffatomen, können bekanntlich durch katalytische Hydroformylierung (technisch meist als Oxoreaktion bezeichnet) der um ein Kohlenstoffatom ärmeren Olefine hergestellt werden. Die Aldehyde werden zum Beispiel als Synthesevorstufen, zur Erzeugung von Carbonsäuren und als Riechstoffe genutzt. Technisch werden sie oft durch katalytische Hydrierung in die entsprechenden Alkohole überführt, die u.a. als Zwischenprodukte für die Herstellung von Weichmachern und Detergentien Einsatz finden.

Verfahren zur Hydroformylierung von Olefinen sind in der Literatur in großer Zahl beschrieben. Die Wahl des Katalysatorsystems und der optimalen Reaktionsbedingungen für die Hydroformylierung sind von der Reaktivität des eingesetzten Olefins abhängig. Der Einfluss der Struktur des eingesetzten Olefins auf dessen Reaktivität in der Hydroformylierung ist z.B. von J. FALBE, *"New Syntheses with Carbon Monoxide* ", Springer Verlag, **1980**, Berlin, Heidelberg, New York, Seite 95 ff, beschrieben.

Als allgemeine Regel gilt, dass die Geschwindigkeit von Hydroformylierungsreaktionen bei konstanten Rahmenbedingungen mit steigender Kohlenstoffzahl und mit zunehmendem Verzweigungsgrad des Olefins abnimmt. So kann die Reaktionsgeschwindigkeit von linearen Olefinen die der verzweigten Isomeren um mehr als eine Zehnerpotenz übersteigen. Zusätzlich Olefine mit terminaler Doppelbindung reagieren deutlich schneller als Isomere mit der Doppelbindung im Innern des Moleküls. Die unterschiedliche Reaktivität von isomeren Octenen wurde zum Beispiel von B. L. HAYMORE, A. van HASSELT, R. BECK, *Annals of the New York Acad. Sci*., **1983**, *415*, 159-175 untersucht. Eine allgemeine Übersicht und weiterführende Literatur geben B. CORNILS, W. A. HERRMANN, *"Applied Homogeneous Catalysis with* *Organometallic Compounds",* Vol. 1&2, VCH, Weinheim, New York, **1996**.

Technische Olefingemische, die als Edukte für die Hydroformylierungssynthese verwendet werden, enthalten oftmals Olefinisomere der verschiedensten Strukturen mit unterschiedlichen Verzweigungsgraden, unterschiedlicher Lage der Doppelbindung und Olefine unterschiedlicher Molmassen. Dies gilt insbesondere für Olefingemische, die durch Di-, Tri- oder weitergehende Oligomerisierung von Olefinen mit 2 bis 8 C-Atomen oder anderen leicht zugänglichen höheren Olefinen bzw. durch Cooligomerisierung der genannten Olefine entstanden sind. Als Beispiele für typische Olefingemische, die technisch für die Hydroformylierung relevant sind, seien Triund Tetrapropen sowie Di-, Tri- und Tetrabutene genannt.

Bei einer technisch durchgeführten Hydroformylierung ist es erwünscht, neben einem hohen Umsatz eine hohe Selektivität zu erreichen, um eine optimale Ausnutzung des Rohstoffes zu gewährleisten. Um einen hohen Umsatz zu erreichen, müssen bei langsam reagierenden Olefinen oft eine relativ lange Reaktionszeit und/oder höhere Reaktionstemperaturen in Kauf genommen werden. Reaktivere Olefine werden dagegen unter den gleichen Reaktionsbedingungen schon in weitaus kürzerer Zeit zu den Aldehyden umgesetzt. Bei der gemeinsamen Hydroformylierung von Gemischen von Olefinen unterschiedlicher Reaktivität führt dies dazu, dass man relativ lange Reaktionszeiten benötigt, um einen ausreichenden Umsatz auch der schwerer oxierbaren Olefine zu erzielen. Die aus den leichter umsetzbaren Olefinen entstehenden Aldehyde werden jedoch relativ schnell gebildet und liegen dann neben den schwerer hydroformylierbaren Olefinen im Reaktor vor. Dies führt zu unerwünschten Neben- und Folgereaktionen der Aldehyde, z. B. zur Hydrierung, zu Kondensationsreaktionen sowie zur Bildung von Acetalen und Halbacetalen. Vor allem wegen der unterschiedlichen Reaktivität der Olefinisomeren ist es also schwierig, bei der Hydroformylierung hohe Umsätze und gleichzeitig hohe Selektivitäten zu erzielen.

Neben der unvorteilhaften Auswirkung auf die Selektivität gibt es zwei weitere Aspekte, die gegen eine gemeinsame Hydroformylierung von Olefingemischen in einer Stufe bis zu hohen Umsätzen sprechen. Zum einen erfordern die relativ langen Reaktionszeiten bei einer vorgegebenen Kapazität (oder Reaktorleistung) relativ große Reaktorvolumina. Dies ist insbesondere deshalb nachteilig, weil es sich bei Hydroformylierungsverfahren um Prozesse handelt, die bei erhöhtem Druck ablaufen und die Investitionskosten für Druckreaktoren mit deren Größe exponentiell ansteigen. Zum anderen ist man in der Steuerung der Produkteigenschaften der Aldehyde, z.B. bestimmt durch das n/i-Verhältnis, eingeschränkt.

Verfahren zur zweistufigen Hydroformylierung von Olefinen sind bekannt. In EP 562 451 und EP 0 646 563 wird die Hydroformylierung von 1- und 2-Buten enthaltenden Mischungen beschrieben, wobei in der ersten Stufe das 1-Buten in einer heterogenen Reaktion, also in einem Mehrphasensystem, gegebenenfalls unter Zusatz eines Phasentransferreagenz oder Lösungsvermittlers umgesetzt wird und in der zweiten Stufe ein homogen gelöster Katalysator zum Einsatz kommt. Gemäß EP 0 562 451 werden in beiden Stufen Rhodium-Katalysatoren eingesetzt, während nach EP 0 646 563 in der ersten Stufe Rhodium- und in der zweiten Stufe Kobaltkatalysatoren verwendet werden. Gemäß EP 0 562 451 wird das in der ersten Stufe nicht umgesetzte Olefin, vorwiegend 2-Buten, in einer zweiten Stufe in homogener Phase und in Gegenwart von Rhodium als Katalysator hydroformyliert. In EP 0 646 563 wird diese Arbeitsweise dahingehend präzisiert, dass das in der ersten Stufe nicht umgesetzte Olefin gasförmig, zusammen mit Kohlenmonoxid, Wasserstoff und durch Hydrierung entstandenem Butan, den Reaktor verlässt. Dieses Gas wird, gegebenenfalls nach Komprimierung, in die zweite Hydroformylierungsstufe geführt. Die Verfahrensweise gemäß dieser beiden Druckschriften ist für die Hydroformylierung höherer Olefine, d. h. mit mehr als 5 C-Atomen, nicht vorteilhaft einsetzbar, weil die nicht umgesetzten Olefine wegen ihrer relativ hohen Siedepunkte nicht mehr mit vertretbarem Aufwand gasförmig aus der ersten Stufe ausgetragen werden können.

In GB 1 387 657 wird eine zweistufige Hydroformylierung beschrieben, bei der das Reaktionsprodukt der ersten Stufe gasförmig ausgetragen wird und nach Auskondensation der Aldehyde bzw. Alkohole das Abgas der ersten Stufe, das nicht umgesetzte Olefine enthält, zum einen Teil in die erste Stufe zurückführt und zum anderen Teil in einen zweiten Reaktor geleitet wird. Dieses Verfahrenskonzept eignet sich für die Hydroformylierung leicht flüchtiger Olefine mit nicht mehr als 5 Kohlenstoffatomen z. B. für Ethylen oder Propylen. Für die Umsetzung höherer Olefine ist es, wie die zuvor erwähnten Verfahren, nicht zweckmäßig, da die Dampfdrücke der Olefine (und die der Aldehyde) zu gering sind und daher zwangsweise in der Flüssigphase gearbeitet werden muss.

In WO 95/08525 wird ein zweistufiges Hydroformylierungsverfahren beschrieben, bei dem das Reaktionsgemisch gasförmig aus der ersten Stufe ausgetragen wird. Nach dem Verfahren sollen Olefine mit 2 bis 20 C-Atomen, insbesondere 2 bis 8 C-Atomen umgesetzt werden können. Die Hydroformylierung ist Rhodium-katalysiert, und der Katalysator ist in beiden Stufen identisch. Das Beispiel beschreibt die Hydroformylierung von Propylen. Höhere Olefine mit mehr als 5 C-Atomen lassen sich, wie bei den vorher beschriebenen Verfahren, wegen der relativ hohen Siedepunkte der Edukte und Produkte nicht technisch vorteilhaft umsetzen. Die Umsetzung in der Gasphase ist daher energetisch ungünstig.

Eine weitere Variante einer zweistufigen Hydroformylierung ist in DE 3 232 557 beschrieben. In der ersten Stufe werden die Olefine unter Verwendung eines Kobaltkatalysators mit Umsätzen von 50-90% hydroformyliert, der Kobaltkatalysator vom Reaktionsgemisch abgetrennt und die gebildeten Aldehyde zusammen mit den nicht umgesetzten Olefinen in eine zweite Hydroformylierungsstufe eingebracht. Der hier eingesetzte ligandmodifizierte Kobaltkatalysator bewirkt nicht nur eine weitere Hydroformylierung der Olefine, sondern gleichzeitig eine Hydrierung der Aldehyde zu den Alkoholen. Außerdem werden die in der ersten Stufe entstandenen Aldehyde den energischen Reaktionsbedingungen der zweiten Stufe ausgesetzt. Dies führt zu Folgereaktionen, insbesondere zu Kondensationsreaktionen unter Bildung von Hochsiedern.

### Aufgabe der Erfindung

Die Aufgabe der Erfindung bestand daher darin, ein Verfahren zur Herstellung von höheren Oxo-Alkoholen aus Olefinen oder Olefingemischen bereitzustellen, das hohe Umsätze mit hohen Selektivitäten verbindet, dementsprechend weniger Neben- und/oder Folgeprodukte erzeugt, sich zudem durch hohe Raum-Zeit-Ausbeuten auszeichnet und mehr Spielraum zur Steuerung der Produkteigenschaften bietet.

Gegenstand der vorliegenden Erfindung ist daher ein Verfahren zur mehrstufigen Kobalt- oder Rhodium-katalysierten Hydroformylierung von Olefinen mit 6 bis 24 Kohlenstoffatomen zu zuerst eine selektive Hydrierung erfolgt, bevor nicht umgesetztes Olefin aus dem Reaktionsprodukt abgetrennt und einer weiteren Hydroformylierung zugeführt wird.

### Aufgabe der Erfindung

Die Aufgabe der Erfindung bestand daher darin, ein Verfahren zur Herstellung von höheren Oxo-Aldehyden bzw. den entsprechenden Alkoholen aus Olefinen oder Olefingemischen bereitzustellen, das hohe Umsätze mit hohen Selektivitäten verbindet, dementsprechend weniger Neben- und/oder Folgeprodukte erzeugt, sich zudem durch hohe Raum-Zeit-Ausbeuten auszeichnet und mehr Spielraum zur Steuerung der Produkteigenschaften bietet.

Gegenstand der vorliegenden Erfindung ist daher ein Verfahren zur mehrstufigen Kobalt- oder Rhodium-katalysierten Hydroformylierung von Olefinen mit 6 bis 24 Kohlenstoffatomen zu Alkoholen, wobei die Olefine
a) in einem Hydroformylierungsschritt bis zu einem Umsatz von 20 bis 98 % hydroformyliert werden,
b) der Katalysator aus dem so erhaltenen flüssigen Reaktoraustrag entfernt wird,
c) das so erhaltene flüssige Hydroformylierungsgemisch in eine Leichtsiederfraktion, enthaltend Olefine und Paraffine und eine Sumpffraktion, enthaltend Aldehyde und/oder Alkohole getrennt wird,
d) die in der Leichtsiederfraktion enthaltenden Olefine in weiteren Verfahrensstufen, umfassend die Verfahrensschritte a, b und c umgesetzt werden und die Sumpffraktionen der Verfahrensschritte c) aller Verfahrensstufen vereinigt werden.

Bevorzugt wird das erfindungsgemäße Verfahren so ausgeübt, dass der flüssige Reaktoraustrag der Hydroformylierungsschritte a) eine homogene Flüssigphase ist. Die Kobalt- oder Rhodium-Katalysatoren werden bevorzugt so eingesetzt, dass sie homogen im flüssigen Reaktoraustrag der Hydroformylierungsschritte a) gelöst sind.

Die Trennung der nicht umgesetzten Olefine von den gebildeten Aldehyden erfolgt nach der Abtrennung überschüssigen Synthesegases und des Katalysators in einem oder mehreren Trennschritten bzw. Destillationsschritten. Die Hydroformylierungsprodukte aus der ersten Verfahrensstufe werden somit nicht nochmals in einer oder mehreren weiteren Stufen den Folgereaktionen begünstigenden Bedingungen einer Hydroformylierungsreaktion unterworfen.

### Beschreibung der Varianten der Erfindung

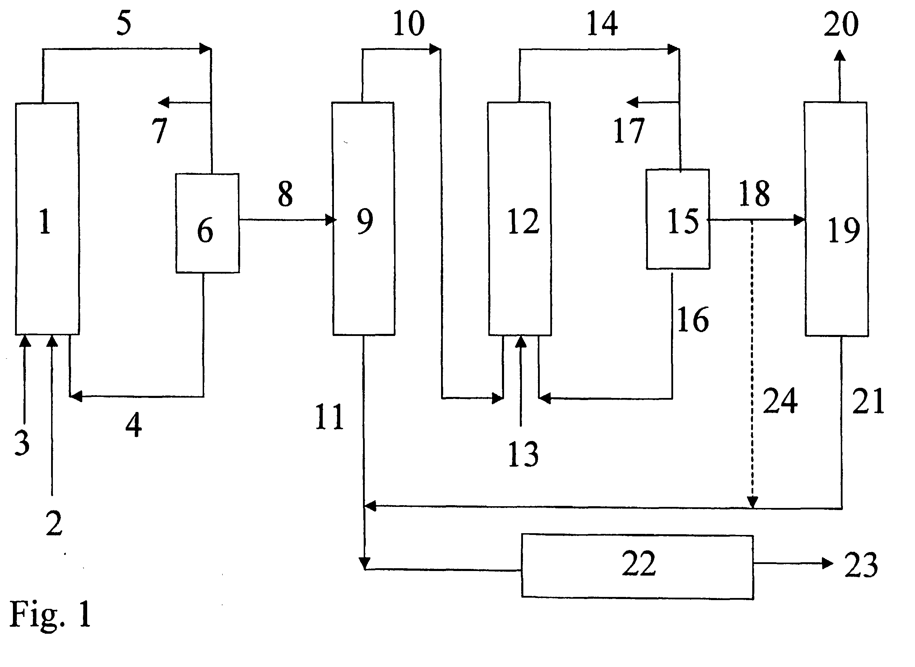

Das erfindungsgemäße Verfahren kann jeweils bevorzugt mit zwei Verfahrensstufen, diskontinuierlich oder kontinuierlich durchgeführt werden. Für eine kontinuierliche Durchführung sind verschiedene Verfahrensvarianten möglich, die exemplarisch als Zweistufenprozess in den Figuren 1 bis 3 dargestellt sind. Im Folgenden werden diese Ausführungsformen als Variante 1, 2 und 3 bezeichnet. Es sei betont, dass die hierbei beschriebenen Verfahrensweisen sinngemäß auch für Verfahren mit mehr als zwei Verfahrensstufen gelten.

Die mittels des erfindungsgemäßen Verfahrens gewonnenen Rohaldehyde, die neben den Verfahrensprodukten Aldehyd und Alkohol noch Formiate, Kondensationsprodukte und weitere Schwersieder enthalten, werden entweder destillativ zur Isolierung des Aldehyds, oder zunächst hydriert und anschließend destillativ zur Isolierung des Alkohols, aufgearbeitet.

### Variante 1

Das Verfahren nach Variante 1 ist als Blockschema in Fig. 1 wiedergegeben. Im ersten Hydroformylierungsreaktor 1 werden das Olefingemisch 3, das Synthesegas 2 (Kohlenmonoxid und Wasserstoff) sowie Katalysatorlösung oder die Vorstufen des Katalysators 4 eingespeist. Das so erhaltene Hydroformylierungsgemisch 5 wird entspannt, das Entspannungsgas 7 (nicht verbrauchtes Synthesegas) wird abgezogen und das entspannte Hydroformylierungsgemisch in der ersten Katalysatorabtrennung 6 vom Katalysator 4 befreit, der, gegebenenfalls nach Ausschleusung eines kleinen Teilstroms und nach Ergänzung durch frischen Katalysator, in den ersten Hydroformylierungsreaktor 1 zurückgeführt wird. Mit Katalysator seien hier auch Vorstufen von Katalysatoren, z. B. Kobalt(II)salzlösungen, bezeichnet. Das vom Katalysator befreite Hydroformylierungsgemisch 8 wird in der Destillationskolonne 9 in die Leichtsieder 10, die überwiegend aus nicht umgesetzten Olefinen bestehen, und Rohaldehyd 11 getrennt. Die Leichtsieder 10, Synthesegas 13 und Katalysatorlösung 16 werden in den zweiten Hydroformylierungsreaktor 12 eingebracht. Der Hydroformylierungsschritt der zweiten Verfahrensstufe kann mit dem gleichen Katalysatorsystem (sowohl Metall als auch Ligand oder deren jeweilige Konzentration) oder mit einem anderen Katalysatorsystem als die erste Stufe betrieben werden. Das Hydroformylierungsgemisch 14 aus dem zweiten Hydroformylierungsreaktor 12 wird wiederum entspannt, und das Entspannungsgas 17 abgezogen. Das entspannte Hydroformylierungsgemisch 14 wird in der zweiten Katalysatorabtrennung 15 vom Katalysator 16 befreit, der wiederum, gegebenenfalls nach Ausschleusung eines kleinen Teilstroms und nach Ergänzung durch frischen Katalysator, in den zweiten Hydroformylierungsreaktor 12 zurückgeführt wird. Das entkatalysierte Hydroformylierungsgemisch 18 kann in der Kolonne 19 in die Leichtsieder 20, die überwiegend aus gesättigten Kohlenwasserstoffen bestehen, und Rohaldehyd 21 aufgetrennt werden. Gegebenenfalls kann ein Teil der Leichtsieder 20 in den Reaktor 12 zurückgefahren werden. (Leitung in Fig. 1 nicht gezeichnet).

Eine weitere Ausgestaltung dieser Verfahrensvariante besteht darin, dass das entkatalysierte Hydroformylierungsgemisch 18 ohne Destillation in der Kolonne 19 zusammen mit dem Rohaldehyd 11 der Hydrierung 22 zugeführt wird (Leitung 24). Die Rohaldehyde 11 und 21 bzw. 11 und 24 werden im Hydrierreaktor 22 mit Wasserstoff zu dem Rohalkohol 23 hydriert, der optional in einer nicht dargestellten Destillation auf reinen Alkohol aufgearbeitet werden kann.

In dieser Ausführungsform der Erfindung weist jede Verfahrensstufe einen Hydroformylierungsschritt a), einen Katalysatorabtrennungsschritt b) und einen Destillationsschritt c) auf, mit der Maßgabe, dass der in b) abgetrennte Katalysator direkt oder nach Aufarbeitung in den Hydroformylierungsschritt a) der jeweiligen Verfahrensstufe zurückgeführt wird.

Optional kann diese Verfahrensvariante auch so durchgeführt werden, dass die letzte Verfahrensstufe keinen Destillationsschritt c) aufweist.

### Variante 2

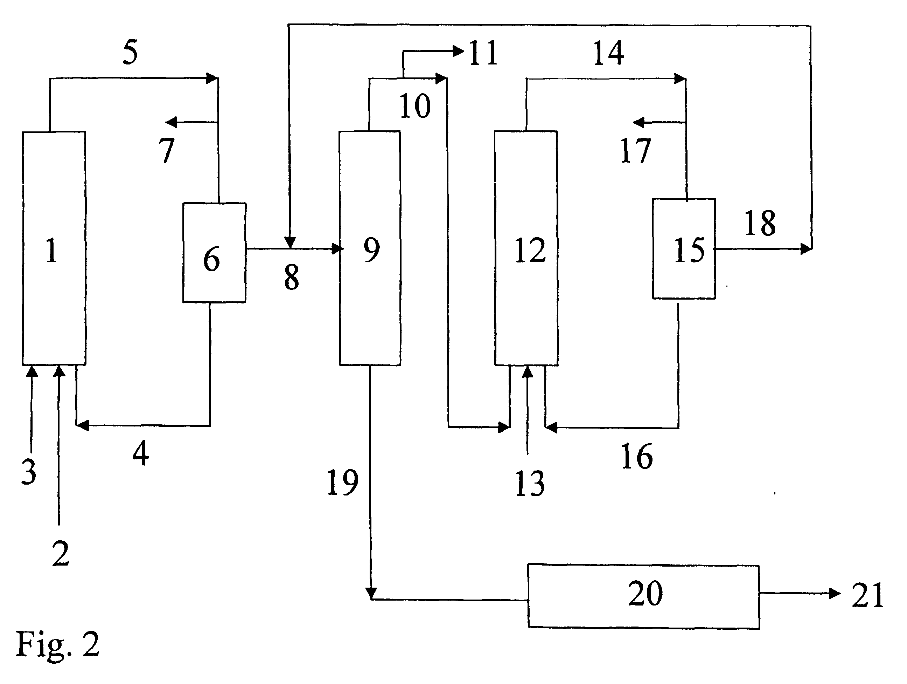

Das Blockschema einer weiteren Verfahrensvariante der Erfindung ist in Fig. 2 dargestellt. Im ersten Hydroformylierungsreaktor 1 werden das Olefingemisch 3, das Synthesegas 2 (Kohlenmonoxid und Wasserstoff) sowie Katalysator 4 oder dessen Vorläufer eingespeist. Das so erhaltene Hydroformylierungsgemisch 5 wird entspannt, das Entspannungsgas 7 (nicht verbrauchtes Synthesegas) wird abgezogen und das entspannte Hydroformylierungsgemisch in der ersten Katalysatorabtrennung 6 vom Katalysator 4 befreit, der, gegebenenfalls nach Ausschleusung eines kleinen Teilstroms und nach Ergänzung durch frischen Katalysator, in den ersten Hydroformylierungsreaktor 1 zurückgeführt wird. Das vom Katalysator befreite Hydroformylierungsgemisch 8 wird in die Destillation 9 geleitet. Dort wird es zusammen mit dem entkatalysierten Hydroformylierungsgemisch 18 aus dem zweiten Hydroformylierungsreaktor 12 in eine Leichtsiederfraktion 10, die die nicht umgesetzten Olefine und inerte Paraffine enthält, und Rohaldehyd 19 getrennt. Die Leichtsieder 10 werden, nach Ausschleusung eines Teilstroms 11 zur Abtrennung von gesättigten Kohlenwasserstoffen (Paraffine) und sonstigen, nicht olefinischen Verbindungen, zusammen mit Synthesegas 13 und Katalysator 16 in den zweiten Hydroformylierungsreaktor 12 geführt. Das so erhaltene Hydroformylierungsgemisch 14 wird entspannt, das Entspannungsgas 17 abgezogen, und das entspannte Hydroformylierungsgemisch in der zweiten Katalysatorabtrennung 15 vom Katalysator 16 befreit, der, gegebenenfalls nach Ausschleusung eines kleinen Teilstroms und nach Ergänzung durch frischen Katalysator, in den zweiten Hydroformylierungsreaktor 12 zurückgeführt wird. Das entkatalysierte zweite Hydroformylierungsgemisch 18 wird mit dem Hydroformylierungsgemisch 8 der ersten Stufe, wie bereits erwähnt, in die Destillationskolonne 9 eingespeist. Der Rohaldehyd 19 wird in der Hydriereinheit 20 mit Wasserstoff zum Rohalkohol 21 hydriert. Dieser Alkohol kann wiederum in einer nicht dargestellten Destillation auf reinen Alkohol aufgearbeitet werden.

Mit Katalysator seien hier auch Vorstufen von Katalysatoren, z. B. Kobalt(II)salzlösungen bezeichnet. Die zweite bzw. jede weitere Verfahrensstufe kann mit dem gleichen Katalysatorsystem (sowohl Metall als auch Ligand oder deren jeweilige Konzentration) oder mit einem anderen System als die erste Verfahrensstufe betrieben werden.

Die Ausschleusung der gesättigten Kohlenwasserstoffe kann anstatt über den Teilstrom 11 auch durch Aufarbeitung eines Teilstroms des vom Katalysator befreiten Hydroformylierungsprodukts 18 erfolgen (nicht dargestellt). Technisch ist dies zum Beispiel durch eine destillative Auftrennung dieses Teilstroms in Leichtsieder, die ausgeschleust werden, und Aldehyde, die in das entkatalysierte Hydroformylierungsgemisch 18 oder den Rohaldehyd 19 zurückgeführt werden, realisierbar.

Diese Ausführungsform der Erfindung weist für jede Verfahrensstufe einen Hydroformylierungsschritt a) sowie einen Katalysatorabtrennungsschritt b) auf, wobei die vereinigten flüssigen Hydroformylierungsgemische, in einem gemeinsamen Destillationsschritt c) in Leichtsieder- und Sumpffraktion getrennt werden, mit der Maßgabe, dass der in den Schritten b) abgetrennte Katalysator direkt oder nach Aufarbeitung in den Hydroformylierungsschritt a) der jeweiligen Verfahrensstufe zurückgeführt wird.

### Variante 3

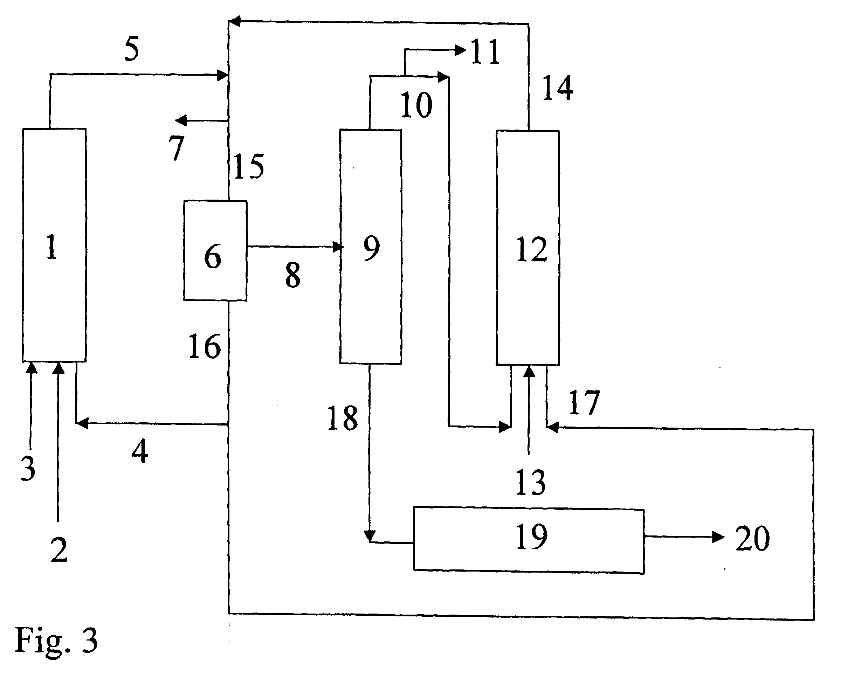

Eine weitere Variante des erfindungsgemäßen Verfahrens ist in Fig. 3 dargestellt. In den ersten Hydroformylierungsreaktor 1 werden das Olefingemisch 3, das Synthesegas 2 (Kohlenmonoxid und Wasserstoff) sowie Katalysatorlösung oder dessen Vorläufer 4 eingespeist. Das so erhaltene Hydroformylierungsgemisch 5 wird zusammen mit dem Hydroformylierungsgemisch 14 aus dem zweiten Hydroformylierungsreaktor 12 als vereinigte Hydroformylierungsausträge 15 entspannt, und das Entspannungsgas 7 (nicht verbrauchtes Synthesegas) abgezogen. In der Katalysatorabtrennung 6 werden die vereinigten Hydroformylierungsausträge vom Katalysator 16 befreit und man erhält ein die gebildeten Aldehyde, Alkohole und nicht umgesetzte Olefine enthaltendes Gemisch 8. Der Katalysator 16 wird, gegebenenfalls nach Ausschleusung einer Teilmenge und Ergänzung durch frischen Katalysator, in die beiden Teilströme 4 und 17 unterteilt. Teilstrom 4 wird in den ersten Hydroformylierungsreaktor 1 und Teilstrom 17 in den zweiten Hydroformylierungsreaktor 12 zurückgefahren. Der entkatalysierte Hydroformylierungsaustrag 8 wird in der Destillationskolonne 9 in die Leichtsieder 10 und den Rohaldehyd 18 aufgetrennt. Die Leichtsiederfraktion 10, die die nicht umgesetzten Olefine enthält, wird, gegebenenfalls nach Ausschleusung einer Teilmenge 11 (zur Abtrennung von gesättigten Kohlenwasserstoffen oder sonstigen nicht olefinischen Verbindungen), zusammen mit Synthesegas 13 und Katalysator 17 in den zweiten Hydroformylierungsreaktor 12 eingeleitet. Der Rohaldehyd 18 wird in der Hydriereinheit 19 mit Wasserstoff zum Rohalkohol 20 hydriert werden. Dieser kann wiederum in einer nicht dargestellten Destillation auf reinen Alkohol aufgearbeitet werden.

Auch bei der Variante 3 ist es möglich, die Ausschleusung von gesättigten Kohlenwasserstoffen über eine separate Aufarbeitung eines Teilstroms des Hydroformylierungsgemisches 14 durchzuführen, zum Beispiel durch destillative Abtrennung der Leichtsieder.

Diese Ausführungsform des erfindungsgemäßen Verfahrens ist dadurch gekennzeichnet, dass die vereinigten Reaktorausträge aller Hydroformylierungsschritte a) nur einen Katalysatorabtrennungsschritt b) und einen Destillationsschritt c) durchlaufen, mit der Maßgabe, dass der in den Verfahrensschritten b) abgetrennte Katalysator direkt oder nach Aufarbeitung aufgeteilt und die Hydroformylierungsschritte a) der einzelnen Verfahrensstufen zurückgeführt wird.

Mit Katalysator sind auch in dieser Variante Vorstufen von Katalysatoren, z. B. Kobalt(II)salzlösungen eingeschlossen.

In dieser Verfahrensvariante muss in allen Hydroformylierungsschritten bzw. Verfahrensstufen der gleiche Katalysator, d. h. Kobalt oder Rhodium als aktives Katalysatormetall verwendet werden. Es ist jedoch möglich, in verschiedenen Verfahrensstufen bzw. deren Hydroformylierungsschritten verschiedene Katalysatorkonzentrationen zu verwenden.

Im erfindungsgemäßen Verfahren ist es möglich, das abgetrennte überschüssige Synthesegas in den Prozess vollständig oder teilweise zurückzuführen. Eine besonders interessante Möglichkeit ergibt sich, wenn man die Hydroformylierungs-Reaktoren bei unterschiedlichen Drücken betreibt. Das Abgas von Reaktoren, die mit höheren Druck als andere betrieben werden, kann bei einem Druck, der über dem Betriebsdruck anderer Reaktoren liegt, abgetrennt werden, so dass es ohne Kompression in den anderen Reaktoren eingesetzt werden kann.

Das gemeinsame Merkmal der Erfindung bzw. den Varianten 1 bis 3 ist die Hydroformylierung von Olefinen oder Olefingemischen in mehreren, vorzugsweise in zwei Stufen, wobei in der ersten Stufe vorwiegend die reaktionsfähigeren Olefine und in den weiteren Stufen vorwiegend die reaktionsträgeren Olefine umgesetzt werden. Ein anderes erfindungswesentliches Merkmal ist die Abtrennung der in den Leichtsiedern enthaltenen nicht umgesetzten Olefine aus dem flüssig ausgetragenen Hydroformylierungsprodukt der ersten Stufe, nach Abtrennung des Katalysators, vorzugsweise durch Destillation. Die wesentlichen Unterschiede zwischen den einzelnen Varianten bestehen im Aufwand zur Aufarbeitung der Reaktionsausträge. Variante 1 ermöglicht durch die getrennt arbeitenden Katalysatorkreisläufe den Einsatz unterschiedlicher Katalysatoren, unterschiedlicher Katalysatorkonzentrationen oder unterschiedlicher Ligandensysteme in den Reaktoren. In Variante 1 garantieren die getrennten Destillationen die beste Abtrennung von im Prozess anfallenden Paraffinen. Es ist aber möglich, mindestens eine der Destillationen einzusparen und die Austräge der verschiedenen Hydroformylierungsreaktoren in nur einem Destillationsschritt aufzutrennen (Variante 2). Eine weitere Verringerung der notwendigen Apparate wird durch die Zusammenlegung der Katalysatorkreisläufe erreicht (Variante 3). Man kann zwar nicht mehr unterschiedliche Katalysatoren in den Verfahrensstufen einsetzen, die Konzentration des Katalysators in den Reaktoren kann aber immer noch durch das Splitverhältnis ( Teilströme 4 und 17 bei einem Zweistufenverfahren gemäß Figur 3) des zurückgeführten Katalysators eingestellt werden. Auch die Reaktionsbedingungen wie Druck, Temperatur etc. bleiben für jeden Hydroformylierungsschritt unabhängig von einander frei wählbar.

Die Reaktoren, in denen die Hydroformylierung durchgeführt wird, können in allen Verfahrensstufen gleich oder verschieden sein. Beispiele für einsetzbare Reaktortypen sind Blasensäulen, Schlaufenreaktoren, Strahldüsenreaktoren, Rührreaktoren und Rohrreaktoren, die zum Teil kaskadiert und/oder mit Einbauten versehen sein können.

### Beschreibung der Edukte, Verfahrensbedingungen und Produkte

Die Edukte für das Verfahren sind Olefine oder Gemische von Olefinen mit 6 bis 24 Kohlenstoffatomen, vorteilhaft mit 6 bis 20 Kohlenstoffatomen, insbesondere mit 8 bis 20 Kohlenstoffatomen sowie mit end- oder innenständigen C-C-Doppelbindungen. Die Gemische können aus Olefinen gleicher, ähnlicher (± 2) oder deutlich unterschiedlicher (> ± 2) C-Zahl bestehen. Als Olefine, die entweder in reiner Form, in einem Isomerengemisch oder in einem Gemisch mit weiteren Olefinen anderer C-Zahl als Edukt eingesetzt werden können, seien beispielsweise genannt: 1-, 2- oder 3-Hexen, 1-Hepten, lineare Heptene mit innenständiger Doppelbindung (2-Hepten, 3-Hepten usw.), Gemische linearer Heptene, 2- oder 3-Methyl-1-hexen, 1-Octen, lineare Octene mit innenständiger Doppelbindung, Gemische linearer Octene, 2-oder 3-Methylhepten, 1-Nonen, lineare Nonene mit innenständiger Doppelbindung, Gemische linearer Nonene, 2-, 3- oder 4-Methyloctene, 1-, 2-, 3-, 4- oder 5-Decen, 2-Ethyl-1-octen, 1-Dodecen, lineare Dodecene mit innenständiger Doppelbindung, Gemische linearer Dodecene, 1-Tetradecen, lineare Tetradecene mit innenständiger Doppelbindung, Gemische linearer Tetradecene, 1-Hexadecen, lineare Hexadecene mit innenständiger Doppelbindung, Gemische linearer Hexadecene. Geeignete Edukte sind weiterhin u. a. das bei der Dimerisierung von Propen anfallende Gemisch isomerer Hexene (Dipropen), das bei der Dimerisierung von Butenen anfallende Gemisch isomerer Octene (Dibuten), das bei der Trimerisierung von Propen anfallende Gemisch isomerer Nonene (Tripropen), das bei der Tetramerisierung von Propen oder der Trimerisierung von Butenen anfallende Gemisch isomerer Dodecene (Tetrapropen oder Tributen), das bei der Tetramerisierung von Butenen anfallende Hexadecen-Gemisch (Tetrabuten) sowie durch Cooligomerisierung von Olefinen mit unterschiedlicher C-Zahl (bevorzugt 2 bis 4) hergestellte Olefingemische, gegebenenfalls nach destillativer Trennung in Fraktionen mit gleicher oder ähnlicher (±2) C-Zahl. Weiterhin können Olefine oder Olefingemische, die durch Fischer-Tropsch-Synthese erzeugt worden sind, eingesetzt werden. Darüber hinaus können Olefine, die durch Olefin-Metathese oder durch andere technische Prozesse hergestellt worden sind, verwendet werden. Bevorzugte Edukte sind Gemische isomerer Octene-, Nonene-, Dodecene- oder Hexadecene, d. h. Oligomere von niedrigen Olefinen, wie n-Butenen, Isobuten oder Propen. Andere ebenfalls gut geeignete Edukte sind Oligomere aus C₅-Olefinen.

Für die Oligomerisierung von Butenen zu im Wesentlichen C₈-Olefinen enthaltenden Gemischen gibt es im Prinzip drei Verfahrensvarianten. Lange bekannt ist die Oligomerisierung an sauren Katalysatoren, wobei technisch z. B. Zeolithe oder Phosphorsäure auf Trägern eingesetzt werden. Hierbei werden Isomerengemische von verzweigten Olefinen erhalten, die im Wesentlichen Dimethylhexene darstellen (WO 92/ 13818). Ein ebenfalls weltweit ausgeübtes Verfahren ist die Oligomerisierung mit löslichen Ni-Komplexen, bekannt als DIMERSOL-Verfahren (B. CORNILS, W. A. HERRMANN, *"Applied Homogeneous Catalysis with Organicmetallic Compounds"; Vol. 1&2, VCH, Weinheim, New York* **1996**). Die dritte Verfahrensvariante ist die Oligomerisierung an Nickel-Festbett-Katalysatoren; das Verfahren hat Eingang in die Literatur als OCTOL-Prozess (Hydrocarbon Process., Int. Ed. (1986) 65 (2. Sect.1) Seite 31 -33) gefunden.

Für die erfindungsgemäße Herstellung eines C₉-Alkoholgemischs, das sich insbesondere für die Darstellung von Weichmachern eignet, wird bevorzugt ein C₈-Olefingemisch, das aus linearen Butenen nach dem OCTOL-Prozess gewonnen worden ist, eingesetzt.

In dem für die Hydroformylierung eingesetzten Synthesegases liegen Kohlenmonoxid und Wasserstoff im Allgemeinen im Molverhältnis von 1:4 bis 4:1 und vorzugsweise im etwa stöchiometrischen Verhältnis vor.

Im Verfahren der Erfindung arbeitet man mit Kobalt- oder Rhodiumkatalysatoren sowie mit oder ohne Komplex-stabilisierenden Zusätzen, wie organischen Phosphinen oder Phosphiten. Es ist möglich, in allen Hydroformylierungsschritten des Verfahrens entweder mit Rhodiumkatalysatoren oder Kobaltkatalysatoren zu arbeiten. Es ist weiterhin möglich, in der ersten Verfahrensstufe im Hydroformylierungsschritt a) einen Kobaltkatalysator (alternativ: Rhodiumkatalysator) und in den Hydroformylierungsschritten der weiteren Verfahrensstufen Rhodiumkatalysatoren (alternativ: Kobaltkatalysatoren) einzusetzen. Es ist ein Vorzug des Verfahrens nach der Erfindung, dass man in den einzelnen Stufen unterschiedliche Katalysatoren verwenden kann, so dass bei mehr als zwei Verfahrensstufen auch mit verschiedenen Katalysatoren gearbeitet werden kann, z. B. Kobalt/Rhodium/Kobalt.

Die Wahl des Katalysators und der Reaktionsbedingungen (Konzentration des Katalysators, Temperatur, Druck, Verweilzeit) hängt u. a. von der Anzahl der Kohlenstoffatome und der Zusammensetzung der Ausgangsolefine ab. Wenn ein hoher Anteil an terminal hydroformyliertem Olefin ein Kriterium für eine hohe Produktqualität ist, so erzielt man beispielsweise bei dem als Di-n-buten bekannten Dimerisierungsgemisch von n-Butenen eine sehr gute Produktqualität bei zufrieden stellender Ausbeute, wenn man bei einem Zweistufenverfahren in beiden Stufen unmodifizierte Kobaltkatalysatoren einsetzt.Verwendet man in der ersten Stufe einen unmodifizierten Kobalt- und in den folgenden Stufen einen unmodifizierten Rhodiumkatalysator, so verbessert sich die Ausbeute, während die Produktqualität etwas zurückgeht. Eine weitere Ausbeuteverbesserung und eine Minderung der Produktqualität treten auf, wenn in allen Stufen unmodifizierte Rhodiumkatalysatoren eingesetzt werden. Wenn ein niedriger Anteil an terminal hydroformyliertem Olefin ein Kriterium für eine hohe Produktqualität ist, so erzielt man beispielsweise bei dem als Di-n-buten bekannten Dimerisierungsgemisch von n-Butenen eine gute Produktqualität bei sehr hoher Ausbeute, wenn man bei einem Zweistufenverfahren in beiden Stufen unmodifizierte Rhodiumkatalysatoren einsetzt. Bei Einsatz von ligandmodifizierten Katalysatoren, insbesondere bei Einsatz von Rhodium und Phosphorliganden, kann zudem über die Wahl des Liganden der Anteil an terminal bzw. nicht terminal hydroformyliertem Olefin in einem weiteren Rahmen beeinflusst werden. Für ein gegebenes Ausgangsolefin lassen sich die optimale Anzahl der Verfahrensstufen sowie in den einzelnen Hydroformylierungsschritten die optimalen Katalysatoren unschwer durch orientierende Versuche ermitteln. Die Katalysatorkonzentrationen können in den einzelnen Stufen gleich oder unterschiedlich sein.

Die Temperaturen und die Drücke in den Hydroformylierungsschritten der verschiedenen Verfahrensstufen können, je nach Katalysator und Olefingemisch, in weiten Grenzen variieren. Da in der ersten Stufe bevorzugt die reaktiveren Olefine reagieren, stellt man in den Hydroformylierungsschritten der weiteren Stufen zweckmäßig energischere Reaktionsbedingungen hinsichtlich Temperatur, Katalysatormenge, Verweilzeit ein.

Die optimalen Bedingungen können, je nach Zielsetzung, von Fall zu Fall variieren: So können zum Beispiel die insgesamt erzielte Raum-Zeit-Ausbeute, die Erhöhung der Selektivität oder die gewünschten Produkteigenschaften ein Optimierungskriterium sein. In der Regel wird die Zusammensetzung des Eduktolefins und die Wahl der Katalysatorsysteme und/oder der Reaktionsbedingungen den Ausschlag geben, welche der möglichen Ausführungsformen des erfindungsgemäßen Verfahrens die ökonomisch optimale ist.

Im erfindungsgemäßen Verfahren werden Olefin-Umsätze in den Hydroformylierungsschritten der einzelnen Verfahrensstufen von 20 bis 98 %, insbesondere von 40 bis 80 %, besonders bevorzugt 50 bis 75 % erhalten (jeweils single pass).

In den auf die erste Verfahrensstufe folgenden Hydroformylierungsschritten a) der weiteren Verfahrensstufen können die Olefine jeweils bis zu einem Umsatz von mindestens 50 %, bevorzugt 55 bis 98 % umgesetzt werden.

Vorteilig bei dem erfindungsgemäßen Verfahren ist, dass in den Hydroformylierungsreaktoren unterschiedliche Reaktionsbedingungen eingestellt werden können. Dies ermöglicht die Anpassung der Hydroformylierungsbedingungen an die Reaktivität des zugeführten Olefingemisches. Zur Minimierung von Folge- und Nebenprodukten ist es zum Beispiel sinnvoll, im ersten Reaktor die reaktionsfreudigen Olefine unter möglichst milden Bedingungen umzusetzen, so dass dort nahezu keine Folge- und Nebenprodukte entstehen. Im folgenden Reaktor wird dann unter gegebenenfalls schärferen Bedingungen das verbleibende Olefingemisch, das überwiegend aus den reaktionsträgen Olefinen besteht, hydroformyliert. Es ist somit möglich, über die unterschiedlichen Reaktionsbedingungen in den Reaktoren die Isomerenverteilung der gebildeten Aldehyde zu beeinflussen.

Rhodium- und Kobalt-katalysierte Hydroformylierungsverfahren unterscheiden sich meistens durch ihre Betriebsparameter. Der Hauptunterschied liegt jedoch in der grundsätzlich unterschiedlichen Katalysatorabtrennung und -rückführung. Im Folgenden werden die beiden Verfahren getrennt erläutert.

### Kobalt-katalysiertes Hydroformylierungsverfahren

Bei der Kobalt-katalysierten Hydroformylierung von Olefinen können unmodifizierte und/oder modifizierte Katalysatoren eingesetzt werden, die für jede Verfahrensstufe gleich oder verschieden sein können. Der Hydroformylierungsprozess in jeder der Kobalt-katalysierten Verfahrensstufen kann nach einem in DE 196 54 340 beschriebenen 1-Stufen-Prozess durchgeführt werden. Nach diesem Verfahren werden die Ausgangsstoffe, die Kobaltsalzlösung, die organische Phase und das Synthesegas, gleichzeitig, vorzugsweise mit Hilfe einer Mischdüse, im Gleichstrom von unten in den Reaktor eingebracht.

Als Kobaltverbindungen werden bevorzugt Kobaltsalze wie Formiate, Acetate oder Salze von Carbonsäuren, die wasserlöslich sind, verwendet. Besonderes bewährt hat sich Kobaltacetat, das als wässrige Lösung mit einem Kobalt-Gehalt von 0,5 bis 3 Gew.-%, vorzugsweise von 1,0 bis 2,0 Gew.-%, gerechnet als Metall, eingesetzt wird.

Die organische Phase enthält das zu hydroformylierende Olefin und gegebenenfalls zusätzlich einen Aldehyd und/oder Alkohol, wobei es sich bei dem Aldehyd oder Alkohol vorzugsweise um die während der Hydroformylierung gebildeten Reaktionsprodukte handelt.

Eine besondere Bedeutung wird bei dem Kobaltkatalysierten Verfahren der Dosierung der Ausgangsstoffe in den Reaktor beigemessen. Die Dosiervorrichtung muss eine gute Phasenvermischung und die Erzeugung einer möglichst hohen Phasenaustauschfläche gewährleisten. Es ist daher bei Kobalt-katalysierter Hydroformylierung vorteilhaft, den Reaktorraum der Hydroformylierungs-Reaktoren durch den Einbau von wenigen, senkrecht zur Fließrichtung des Reaktanden- und Produktenstromes angeordneten Lochblechen (Mindestzahl =1) zu unterteilen. Durch die Reaktorkaskadierung wird die Rückvermischung gegenüber der einfachen Blasensäule stark vermindert und das Strömungsverhalten dem eines Rohreaktors angenähert. Diese verfahrenstechnische Maßnahme hat zur Folge, dass sowohl die Ausbeute als auch die Selektivität der Hydroformylierung verbessert werden.

Werden erfindungsgemäß Hydroformylierungsschritte mit Kobalt-Katalysator verwendet, so werden diese bei Temperaturen von 100 bis 250 °C und unter Drücken von 100 bis 400 bar betrieben. Besonderes bewährt haben sich Temperaturen von 140 bis 210 °C und Synthesegas-Drücke von 200 bis 300 bar. Das Volumenverhältnis vom Kohlenmonoxid zum Wasserstoff im Synthesegas liegt im Allgemeinen zwischen 2 : 1 und 1 : 2, insbesondere im Volumenverhältnis von 1 : 1. Das Synthesegas wird vorteilhaft im Überschuss, zum Beispiel bis zu dem Dreifachen der stöchiometrischen Menge, verwendet.

Die Hydroformylierung von Olefinen wird mittels Kobalt-Katalyse in der ersten Verfahrensstufe, in der die reaktiveren Olefine umgesetzt werden, bei Temperaturen zwischen 140 bis 195 °C, vorzugsweise bei 160 bis 185 °C durchgeführt. Olefin-Umsätze zwischen 20 und 90 %, vorzugsweise zwischen 50 und 80 %, werden in dieser Verfahrensstufe angestrebt.

Der Produktaustrag wird nach Verlassen des Reaktors der ersten Verfahrensstufe bzw. des ersten Hydroformylierungsschritts auf 10 bis 15 bar entspannt und in die Entkobaltung (Katalysatorabtrennung, 6 in Fig. 1) geleitet. Im Entkobaltungsschritt wird der Produktaustrag (organische Phase) in Gegenwart von "Prozesswasser" mit Luft oder Sauerstoff bei Temperaturen von 130 bis 190 °C von Kobalt-Carbonylkomplexen befreit. Die Entkobaltungsverfahren sind gut bekannt und in der Literatur ausführlich, wie z. B von J. FALBE, in "New Syntheses with Carbon Monoxide", Springer Verlag (1980), Berlin, Heidelberg, New York, Seite 158 ff, beschrieben.

Die Entkobaltung wird vorzugsweise in einem mit Füllkörpern, wie z.B. Raschig-Ringen, befüllten Druckbehälter, in dem möglichst hohe Phasenaustauschfläche erzeugt wird, durchgeführt. Die kobaltfreie organische Produktphase wird in einem nachgeschalteten Trennbehälter von der wässrigen Phase getrennt. Die wässrige Phase, das "Prozesswasser", die das zurückextrahierte, aus der organischen Phase wiedergewonnene Kobalt in Form von Kobaltacetat/formiat enthält, wird ganz oder nach Ausschleusung eines geringen Anteils in den Oxo-Reaktor der jeweiligen Verfahrensstufe zurückgeführt und vorzugsweise als Ausgangsstoff für die in-situ Herstellung der Kobalt-Katalysatorkomplexe verwendet.

Bevorzugt werden Vorcarbonylierung, Katalysatorextraktion und die eigentliche Hydroformylierung gemäß DE 196 54 340 in einem Reaktor durchgeführt. Es ist auch möglich, diese Verfahrensstufen apparativ voneinander zu trennen.

Der organische Reaktoraustrag, der die nicht umgesetzten Olefine, Aldehyde, Alkohole, Ameisensäureester und Hochsieder enthält, wird nach dem Hydroformylierungsschritt und der Katalysatorabtrennung einem Destillationsschritt zugeführt. Hier wird der vom Kobalt-Katalysator und überschüssigen Synthesegas befreite Reaktoraustrag destillativ in die rohen Aldehyde/Alkohole (Sumpffraktion) und eine Leichtsiederfraktion getrennt, die, je nach Verfahrensvariante und Bedingungen des Hydroformylierungsschritts, hauptsächlich aus den nicht umgesetzten, weniger reaktiven Olefinen und/oder durch Hydrierung der Olefine entstandenen Paraffinen besteht.

Die von den Wertprodukten im Destillationsschritt befreiten nicht umgesetzten Olefine werden anschließend in den Hydroformylierungsschritt der nächsten Verfahrensstufe geleitet.

Nach dem erfindungsgemäßen Verfahren wird die Kobalt-katalysierte Hydroformylierung in den auf die erste Stufe folgenden, weiteren Verfahrensstufen bzw. Hydroformylierungsschritten bei Temperaturen von 160 bis 220 °C, vorzugsweise von 175 bis 195 °C durchgeführt. Hier werden Olefin-Umsätze von mindestens 50 %, vorzugsweise zwischen 50 und 95 %, vorzugsweise zwischen 55 und 98 %, angestrebt.

Das erfindungsgemäße, mehrstufige Verfahren bietet die Möglichkeit, durch Anpassung der Reaktionsbedingungen, zum Beispiel durch Wahl von niedrigen Kobalt-Konzentrationen, den Olefin-Umsatz in der ersten Stufe auf den angestrebten Wert zu bringen. In den folgenden Stufen, wo die langsamer reagierenden Olefine umgesetzt werden, können dann die Reaktionsbedingungen verschärft werden, zum Beispiel durch Erhöhung der Katalysatorkonzentration.

Die erfindungsgemäßen Verfahrensstufen mit Kobalt-Katalysator sind insbesondere für die Hydroformylierung von Gemische isomerer Olefine geeignet, die durch Oligomerisierung von Propen und Butenen hergestellt werden. Zu den typischen Oligomerisaten, die vorzugsweise als Rohstoffbasis für die Hydroformylierung nach dem neuen Verfahren einsetzbar sind, zählen Di-, Tri- und Tetra-Propen sowie Di-, Tri-, und Tetra-Buten.

### Rhodium-katalysierte Hydroformylierung

Bei Rhodiumkatalysierten Hydroformylierungsverfahren können modifizierte und/oder unmodifizierte Katalysatoren eingesetzt werden, die für jeden Rhodium-katalysierten Hydroformylierungsschritt gleich oder verschieden sein können.

Diese Rhodiumkatalysatoren können in Form ihrer aktiven Komplexe in den Prozess eingebracht werden, technisch ist es in der Regel aber einfacher, die aktiven Katalysatoren in situ aus stabilen, leicht lagerbaren Rhodiumverbindungen zu generieren. Geeignete Rhodiumverbindungen dafür sind zum Beispiel Rhodium(II)- und Rhodium(III)-salze, wie Rhodium(III)chlorid, Rhodium(III)nitrat, Rhodium(III)sulfat, Kalium-Rhodiumsulfat, Rhodium(II)- bzw. Rhodium(III)-carboxylat, Rhodium(II)- und Rhodium(III)acetat, Rhodium(II)octanoat, Rhodium(II)nonanoat, Rhodium(III)oxid, Salze der Rhodium(III)säure, Trisammoniumhexachlororhodat(III). Weiterhin eignen sich Rhodiumkomplexe, wie Rhodiumbiscarbonylacetylacetonat, Acetylacetonatobisethylenrhodium(I). Besonders geeignet sind Rhodiumacetat, Rhodiumoctanoat und Rhodiumnonanoat.

Im Allgemeinen wird ungefähr 1 bis 500 und vorzugsweise 3 bis 50 Mol Ligand pro Mol Rhodium zugesetzt. Frischer Ligand kann zu jedem Zeitprodukt der Reaktion zugesetzt werden, um die Konzentration an freiem Liganden konstant zu halten.

Die Konzentration des Rhodiums im Hydroformylierungsreaktor liegt zwischen 1 ppm und 500 ppm, vorzugsweise zwischen 5 ppm und 200 ppm.

Die Wahl der zugesetzten Liganden ist im erfindungsgemäßen Verfahren nicht beschränkt, sondern hängt vom eingesetzten Olefin und von den erwünschten Produkten ab. Bevorzugte Liganden sind Liganden die Stickstoff-, Phosphor-, Arsen- oder Antimonatome enthalten, besonders bevorzugt sind Phosphorliganden. Die Liganden können ein oder mehrzähnig sein, bei chiralen Liganden kann sowohl das Racemat als auch ein Enantiomer oder Diastereomer eingesetzt werden. Als Phosphorliganden sind insbesondere Phosphine, Phosphinoxide, Phosphite, Phosphonite und Phosphinite zu nennen. Beispiele für Phosphine sind Triphenylphosphin, Tris(p-tolyl)phosphin, Tris(m-tolyl)phosphin, Tris(o-tolyl)phosphin, Tris(pmethoxyphenyl)phosphin, Tris(p-fluorphenyl)phosphin, Tris(p-chlorphenyl)phosphin, Tris(pdimethylaminophenyl)phosphin, Ethyldiphenylphosphin, Propyldiphenylphosphin, t-Butyldiphenylphosphin, n-Butyldiphenylphosphin, n-Hexyldiphenylphosphin, c-Hexyldiphenylphosphin, Dicyclohexylphenylphosphin, Tricyclohexylphosphin, Tricyclopentylphosphin, Triethylphosphin, Tri-(1-naphthyl)phosphin, Tri-2-furylphosphin, Tribenzylphosphin, Benzyldiphenylphosphin, Tri-n-butylphosphin, Tri-i-butylphosphin, Tri-t-butylphosphin, Bis(2-methoxyphenyl)phenylphosphin, Neomenthyldiphenylphosphin, die Alkali-, Erdalkali-, Ammonium- oder andere Salze sulfonierter Triphenylphosphine wie Tris(msulfonylphenyl)phosphin, (m-Sulfonylphenyl)diphenylphosphin; 1,2-Bis(dicyclohexylphosphino)ethan, Bis(dicyclohexylphosphino)methan, 1,2-Bis(diethylphosphino)ethan, 1,2-Bis(2,5-diethylphospholano)benzol [Et-DUPHOS], 1,2-Bis(2,5-diethylphospholano)ethan [Et-BPE], 1,2-Bis(dimethylphosphino)ethan, Bis(dimethylphosphino)methan, 1,2-Bis(2,5-dimethylphospholano)benzol [Me-DUPHOS], 1,2-Bis(2,5-dimethylphospholano)ethan [Me-BPE], 1,2-Bis(diphenylphosphino)benzol, 2,3-Bis(diphenylphosphino)bicyclo[2.2.1]hept-5-en [NORPHOS], 2,2'-Bis(diphenylphosphino)-1,1'-binaphthyl [BINAP], 2,2'-Bis(diphenylphosphino)-1,1'-bipenyl [BISBI], 2,3-Bis(diphenylphosphino)butan, 1,4-Bis(diphenylphosphino)-butan, 1,2-Bis(diphenylphosphino)ethan, Bis(2-diphenylphosphinoethyl)phenylphosphin, 1,1'-Bis(diphenylphosphino)ferrocen, Bis(diphenylphosphino)methan, 1,2-Bis(diphenylphosphino)propan, 2,2'-Bis(di-p-tolylphosphino)-1,1'-binaphthyl, O-Isopropyliden-2,3-dihydroxy-1,4-bis(diphenylphosphino)butan [DIOP], 2-(Diphenylphosphino)-2'-methoxy-1,1'-binaphthyl, 1-(2-Diphenylphosphino-1-naphthayl)isochinolin, 1,1,1 -Tris(diphenylphosphino)ethan, Tris(hydroxypropyl)phosphin.

Ein besonders bevorzugt eingesetztes Phosphin ist Triphenylphosphin.

Beispiele für Phosphite sind Trimethylphosphit, Triethylphosphit, Tri-n-propylphosphit, Tri-ipropylphosphit, Tri-n-butylphosphit, Tri-i-butylphosphit, Tri-t-butylphosphit, Tris(2-ethylhexyl)phosphit, Triphenylphosphit, Tris(2.4-di-t-butylphenyl)phosphit, Tris(2-t-butyl-4-methoxyphenyl)phosphit, Tris(2-t-butyl-4-methylphenyl)phosphit, Tris(p-kresyl)phosphit. Außerdem sterisch gehinderte Phosphitliganden, wie sie unter anderem in EP 155 508, US 4 668 651, US 4 748 261, US 4 769 498, US 4 774 361, US 4 835 299, US 4 885 401, US 5 059 710, US 5 113 022, US 5 179 055, US 5 260 491, US 5 264 616, US 5 288 918, US 5 360 938, EP 472 071, EP 518 241 und WO 97/20795 beschriebenen werden. Bevorzugt eingesetzt werden mit jeweils 1 oder 2 Isopropyl- und/oder tert.-Butylgruppen an den Phenylringen, vorzugsweise in ortho-Position zur Phosphitestergruppierung, substituierte Triphenylphosphite.

Beispiele für Phosphonite sind Methyldiethoxyphosphin, Phenyldimethoxyphosphin, Phenyldiphenoxyphosphin, 6-Phenoxy-6H-dibenz[c,e][1,2]oxaphosphorin und dessen Derivate, in denen die Wasserstoffatome ganz oder teilweise durch Alkyl-, Arylreste oder Halogenatome ersetzt sind und Liganden die in den Patenten WO 9843935, JP 09-268152 und DE 198 10 794 und in den deutschen Patentanmeldungen DE 199 54 721 und DE 199 54 510 beschrieben werden.

Gängige Phosphinitliganden sind unter anderem in US 5 710 344, WO 95 06627, US 5 360 938, JP 07082281 beschrieben. Beispiele hierfür sind Diphenyl(phenoxy)phosphin und dessen Derivate, in denen die Wasserstoffatome ganz oder teilweise durch Alkyl-, Arylreste oder Halogenatome ersetzt sind, Diphenyl(methoxy)phosphin, Diphenyl(ethoxy)phosphin usw.

Rhodium-katalysierte Hydroformylierungen werden in der Regel bei Drücken von 1 bis 300 bar durchgeführt, vorzugsweise bei Drücken von 15 bis 270 bar. Der angewendete Druck hängt von der Struktur der Einsatzolefine, dem eingesetzten Rhodium-Katalysator und dem gewünschten Effekt ab. So können beispielsweise α-Olefin bei Drücken unter 64 bar mit hohen Raum-Zeit-Ausbeuten zu den entsprechenden Aldehyden umgesetzt werden. Bei Olefinen mit innenständigen Doppelbindungen, insbesondere bei verzweigten Olefinen, sind dagegen höhere Drücke zweckmäßig.

Die Temperaturen für Rhodium-katalysierte Hydroformylierungen liegen im Allgemeinen im Bereich von 40°C bis 180°C, bevorzugt bei 60°C bis 135°C. Bei Temperaturen über 100°C hat man den technischen Vorteil, die Abwärme der Reaktion zur Erzeugung von Dampf nutzen zu können.

Nach der Hydroformylierung wird der größte Teil des Synthesegases durch Druckentspannung entfernt. Aus dem flüssigem Reaktionsaustrag wird der Katalysator destillativ abgetrennt (Katalysatorabtrennung z. B. 6 und 15 in Fig. 1). Der Katalysator und gegebenenfalls zugesetzte Liganden, Stabilisatoren usw. verbleiben im/als Destillationsrückstand. Es daher ist vorteilhaft, ein hochsiedendes (höher siedend als Produkte und Edukte), inertes Lösungsmittel einzusetzen, in dem sich der Katalysator löst. Der im hochsiedenden Lösungsmittel gelöste Katalysator kann dann direkt in die Reaktoren zurückgefahren werden. Besonders vorteilhaft ist es, als hochsiedendes Lösungsmittel die im Prozess gebildeten hochsiedenden Nebenprodukte einzusetzen. Andere geeignete Lösungsmittel sind hochsiedene Ester, wie 2,2,4-Trimethylpentandiol-1,3-monoisobutyrat, das als Texanol im Handel ist.

Für die technische Ausführung der destillativen Katalysatorabtrennung sind verschiedene Verfahrensweisen anwendbar. Bevorzugt ist die Abtrennung der Katalysatorlösung über Fallfilm-, Kurzstrecken- oder Dünnschichtverdampfer oder Kombinationen aus diesen Apparaten. Der Vorteil einer solchen Kombination kann zum Beispiel darin liegen, in einem ersten Schritt noch gelöstes Synthesegas sowie ein Teil der Produkte und der noch vorhandenen Ausgangsolefine (zum Beispiel in einem Fallfilmverdampfer) abzutrennen, um dann in einem zweiten Schritt (zum Beispiel in einem Dünnschichtverdampfer), die endgültige Abtrennung des Katalysators vorzunehmen.

Da die Oxierung von Olefinen eine exotherme Reaktion ist, muss die entstehende Wärme aus den Reaktoren abgeführt werden, um die Temperatur im Reaktor zu begrenzen. Zu hohe Temperaturen bewirken in der Regel eine vermehrte Bildung von Nebenprodukten und die Desaktivierung des Katalysators. Oft ist auch ein möglichst isothermer Verlauf deshalb erwünscht, weil die Reaktionstemperatur direkten Einfluss auf die Produktzusammensetzung (z. B. das n/i-Verhältnis) haben kann.

Die Wärmeabfuhr ist über verschiedene technische Ausführungen möglich, zum Beispiel über die Reaktorwand, eingebaute Kühler usw. Technisch vorteilhaft ist es, den Aufwand für die Abfuhr der Wärme gering zu halten. Durch die unterschiedliche Reaktionsgeschwindigkeit bei Einsatz von Olefinmischungen kann es aber, insbesondere in der ersten Stufe, infolge der Exothermie zu einer erheblichen Wärmeentwicklung kommen, da hier bevorzugt die leicht oxierbaren Komponenten abreagieren. Das erfindungsgemäße Verfahren bietet dann die Möglichkeit, durch Anpassung der Reaktionsbedingungen, zum Beispiel eine niedrige Katalysatorkonzentration oder Zugabe eines inerten Lösungsmittel, die Wärmeentwicklung vor allem in der ersten Verfahrensstufe in technisch einfach beherrschbarem Rahmen zu halten

### Aufarbeitung der Katalysator-freien Hydroformylierungsgemische

Die vom Katalysator und überschüssigen Synthesegas befreiten Reaktorausträge werden, wie in den Figuren 1-3 gezeigt, getrennt oder gemeinsam destillativ in die rohen Aldehyde und eine Leichtsiederfraktion getrennt. Die Leichtsieder bestehen je nach Verfahrensvariante und Verfahrensstufe hauptsächlich aus nicht umgesetzten Olefinen oder durch Hydrierung der Olefine entstandenen Paraffinen. Das Sumpfprodukt enthält neben Aldehyden und Alkoholen auch hochsiedende Nebenprodukte wie Formiate, Acetale, gesättigte und ungesättigte Ether, Ester, Carbonsäuren sowie Kondensationsprodukte. Man kann die vom Katalysator befreiten Hydroformylierungsausträge getrennt in einer oder mehreren Destillationen (Variante 1) oder in einer gemeinsamen Destillation (Varianten 2 und 3) in Leichtsieder und Rohaldehyd trennen. Die Destillationsbedingungen hängen von den Siedepunkten der Komponenten und damit primär von der Molmasse der Olefine und Aldehyde ab. Sie sind so zu wählen, dass keine größeren Mengen an Nebenprodukten während der Destillation gebildet werden. Da diese hauptsächlich auf Reaktionen der Aldehyde bei erhöhten Temperaturen zurückgehen, kann die Destillation unter vermindertem Druck durchgeführt und so die Temperaturen in der Kolonne niedrig gehalten werden. Es ist aber auch möglich, die Destillation bei Normaldruck durchzuführen.

Wenn die Reaktionsausträge der Hydroformylierungsschritte in getrennten Destillationen aufgearbeitet werden (Variante 1), werden die Leichtsieder der ersten Destillation in die folgende Verfahrensstufe (allgemein: die Leichtsieder einer Stufe in die Nächste) geführt und die Leichtsieder der letzten Destillation ausgeschleust, gegebenenfalls auch zum Teil in die vorherige Hydroformylierungsstufe zurückgeführt. Wenn die Reaktionsausträge verschiedener Verfahrensstufen gemeinsam aufgearbeitet werden (Variante 2 und 3), wird zweckmäßig ein Teil der Leichtsieder vor dem Eintritt in die letzte Verfahrensstufe oder durch Aufarbeitung eines Teilstroms des Austrags der letzten Stufe ausgeschleust, um den Anteil der Paraffine im Kreislauf auf einer akzeptablen Höhe zu halten.

Es ist somit möglich, die Paraffine aus mindestens einer Leichtsiederfraktion ganz oder teilweise auszuschleusen.

Neben diesen auch in der Beschreibung der Varianten 1 bis 3 aufgeführten Möglichkeiten, Leichtsieder und insbesondere Paraffine aus dem Prozess auszuschleusen, kommen noch weitere in Betracht. Wird die Abtrennung des Katalysators und gegebenenfalls auch die Destillation unter vermindertem Druck durchgeführt, wird ein Teil der Leichtsieder und allerdings auch des Produktes über das Vakuumsystem aus dem Prozess entfernt. Nach Kondensation kann dieser Teil verworfen werden, von einer ausreichenden Menge an lohnt eine (teilweise) Rückführung in den Prozess. Auch über das abgetrennte überschüssige Synthesegas wird, je nach Betriebsbedingungen, ein Teil an Leichtsiedern und Produkten ausgetragen, den man (zum Beispiel durch Kondensation) abtrennen und ggf. zurückführen oder aufarbeiten kann.

Die rohen Aldehyde werden in üblicher Weise in gasförmiger oder flüssiger Phase zu den Alkoholen (Zielprodukte) hydriert.

Es werden entweder die vereinigten Sumpffraktionen aus den Destillationsstufen c) oder bei Verzicht auf den Destillationsschritt c) der letzten Verfahrensstufe die vereinigten Sumpffraktionen und der Austrag des letzten Katalysatorabtrennungsschritts b) des Verfahrens hydriert.

Man kann zur Hydrierung z. B. Kupfer/Nickel-, Kupfer/Chrom-, Kupfer/Chrom/Nickel-, Zink/Chrom-, Nickel/Molybdän-Katalysatoren verwenden. Die Katalysatoren können trägerfrei sein, oder die hydrieraktiven Stoffe bzw. ihre Vorläufer können auf Träger, wie beispielsweise Siliciumdioxid oder Aluminiumdioxid, aufgebracht sein.

Bevorzugte Katalysatoren, an denen die Hydroformylierungsgemische hydriert werden, enthalten, jeweils 0,3- 15 Massen-% Kupfer und Nickel sowie als Aktivatoren 0,05 - 3,5 Massen-% Chrom und vorteilhaft 0,01 - 1,6 Massen-%, vorzugsweise 0,02 - 1,2 Massen-% einer Alkalikomponente auf einem Trägermaterial, vorzugsweise Aluminiumoxid und Siliciumdioxid. Die Mengenangaben beziehen sich auf den noch nicht reduzierten Katalysator. Die Alkalikomponente ist optional.

Die Katalysatoren werden vorteilhaft in einer Form eingesetzt, in der sie einen geringen Strömungswiderstand bieten, z. B. in Form von Granalien, Pellets oder Formkörpern, wie Tabletten, Zylindern, Strangextrudate oder Ringen. Sie werden zweckmäßig vor ihrem Einsatz aktiviert, z. B. durch Erhitzen im Wasserstoffstrom.

Die Hydrierung, bevorzugt eine Flüssigphasenhydrierung, wird im Allgemeinen unter einem Gesamtdruck von 5 bis 30 bar durchgeführt, insbesondere zwischen 15 und 25 bar durchgeführt. Eine Hydrierung in der Gasphase kann auch bei niedrigeren Drücken durchgeführt werden, mit entsprechend großen Gasvolumina. Werden mehrere Hydrierungsreaktoren eingesetzt, können die Gesamtdrücke in den einzelnen Reaktoren innerhalb der genannten Druckgrenzen gleich oder verschieden sein.

Die Reaktionstemperaturen liegen bei Hydrierung in flüssiger oder gasförmiger Phase in der Regel zwischen 120 und 220 °C, insbesondere zwischen 140 und 180 °C.
Beispiele für solche Hydrierungen sind in den Patentanmeldungen DE 198 42 369 und DE 198 42 370 beschrieben.

Nach der Hydrierung werden die so erhaltenen Reaktionsgemische durch Destillation aufgearbeitet. Gegebenenfalls abgetrennte Olefine können in die Hydroformylierungsstufe zurückgeführt werden.

Die folgenden Beispiele sollen die Erfindung erläutern, jedoch nicht in ihrem Anwendungsbereich einschränken, wie er durch die Patentansprüche definiert ist.

### Beispiel 1

### Umsetzung von Octen in zwei Stufen mit verschiedenen Katalysatorliganden

In einem 11 Autoklav wurden 100 g 1-Octen (>98%, GC) bei 85°C unter 20 bar Synthesegasdruck umgesetzt. Der Rhodiumkatalysator wurde in situ aus Rhodiumoctanoat und Ligand 1 generiert.

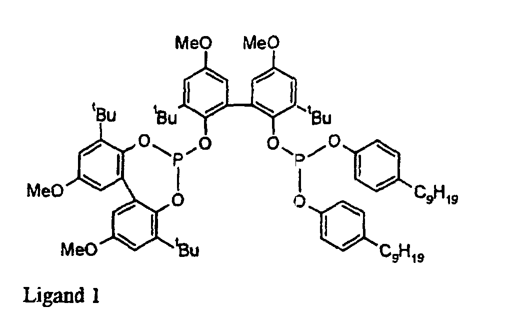

Als inertes, hochsiedendes Lösungsmittel wurden der Reaktion 200 ml Texanol (2.2.4-Trimethylpentandiol-1.3-monoisobutyrat) zugegeben. Die Rhodiumkonzentration wurde auf 40 ppm (bezogen auf die Gesamtmasse) eingestellt, das Phosphor zu Rhodium-Verhältnis (P/Rh) betrug 20/1. Der Umsatz des Olefins wurde über die Menge an aufgenommenem Synthesegas verfolgt. Nachdem ein Umsatz von etwa 90% erreicht worden war, wurde fast keine Gasaufnahme mehr registriert und der Versuch abgebrochen. Laut GC Analyse lag der Umsatz bei 91%, der gebildete Aldehyd bestand zu 95% aus Nonanal. Die Analyse des Restolefins ergab nur Spuren an 1-Octen; Hauptbestandteile waren 2-Octen, 3-Octen und 4-Octen, die durch Isomerisierung des 1-Octen entstanden waren.

Der Versuch wurde 6 mal durchgeführt, die Austräge vereinigt und destilliert. Dabei wurden 43 g Octengemisch erhalten. Diese wurden, gelöst in 100 ml Texanol, bei 120°C und 50 bar Synthesegasdruck in einem 500 ml Autoklaven erneut hydroformyliert. Die Rhodiumkonzentration betrug 40 ppm, als Ligand wurde Tris(2.4-ditert.butylphenyl)phosphit (P/Rh 20/1) zugegeben. Bei dieser Umsetzung wurde ein quantitativer Umsatz des Olefins erreicht (GC).

Das Beispiel zeigt, dass das in der ersten Stufe eingesetzte Katalysatorsystem eine hohe n/iso-Selektivität aufweist, aber nur eine geringe Aktivität für die Hydroformylierung von Octenen mit innenständiger Doppelbindung, wie sie in der ersten Stufe durch Isomerisierung des eingesetzten n-Octens gebildet werden (vgl. P. W. N. M. van Leuwen et al. , Organometallics 1996, 15, 835-847). Diese können jedoch in einer zweiten Stufe unter anderen Versuchsbedingungen umgesetzt werden. Man erreicht also einerseits hohe Selektivität für das erwünschte geradkettige Nonanal und andererseits eine verbesserte Gesamtausbeute bezogen auf den Einsatzstoff.

### Beispiel 2

### Hydroformylierung von Di-n-buten in zwei Stufen mit verschiedenen Katalysatoren

In einem 31 Rührautoklaven wurden ca. 1 000 g Kobaltacetat-haltiges Wasser (Kobaltgehalt ca. 1 Massen-%, gerechnet als Metall) vorgelegt. Unter Rühren (1 000 Upm) wurde das Gemisch unter einen Synthesegasdruck von 280 bar gesetzt und die Temperatur auf 170°C geregelt. Nach 7h wurde auf 60°C abgekühlt und auf 100 bar entspannt. Dann wurden 600g Di-n-buten (Hauptbestandteile 14 % Octene, 60 % 3-Methylheptene, 26 % 3,4-Dimethylhexene) zugegeben. Nach 10-minütigem Rühren (1 000 Upm) blieb das Gemisch 15 Minuten stehen. Die wässrige Phase wurde abgetrennt. Die Di-n-buten-Phase enthielt Kobaltcarbonyle in einer Konzentration von 0,019 Massen-% berechnet als Kobalt. Diese Lösung wurde bei 170°C und 280 bar Synthesegasdruck umgesetzt. Über die Menge des aufgenommenen Synthesegases wurde der Umsatz bestimmt. Bei 70 % Umsatz wurde die Reaktion abgebrochen. Nach Abkühlen auf 80°C und Entspannung wurde das Reaktionsgemisch durch Zugabe von 5 Gew.-%iger wässriger Essigsäure in Gegenwart von Luft entkobaltet. Die entkobaltete organische Phase wurde durch Destillation in die Fraktionen Restolefin/geringer Anteil Paraffin, Aldehyd/Alkohole und Hochsieder aufgetrennt.

Das Restolefin (175 g, Hauptbestandteile ca. 4 % Octene, 52 % 3-Methylheptene, 44 % 3.4-Dimethylhexene) wurde anschließend in einer Rhodium-katalysierten Reaktion analog zu Beispiel 1 umgesetzt. Als inertes Lösungsmittel wurden 200 g Texanol (2,2,4-Trimethylpentandiol-1.3-monoisobutyrat) zugesetzt, die Rhodiumkonzentration auf 200 ppm Rh eingestellt, das molare Verhältnis Ligand (Tris(2,4-ditert.butylphenyl)phosphit) zu Rhodium betrug 20/1. Der Druck betrug konstant 50 bar, die Temperatur 130°C.

Nach 6 Stunden wurde der Autoklav abgekühlt, entspannt, und der Austrag destillativ in die Fraktionen Restolefin/geringer Teil Paraffin, Aldehyde / Alkohole und Hochsieder aufgetrennt. Die vereinigten Aldehyd-/ Alkoholfraktionen aus den beiden Reaktionen wurden über Raney-Nickel zu den Alkoholen hydriert. Die Ausbeute an Alkohol über beide Hydroformylierungsstufen und Hydrierung betrug 87%.

Erfindungsgemäß wird somit in einem zweistufigen Verfahren eine höhere Ausbeute als in einem einstufigen Verfahren (Vergleichsbeispiel 6) erreicht.

### Beispiel 3 (Verbesserung des Umsatzes, Verringerung der Nebenprodukte)

Der Versuch wurde in einer Versuchsanlage, bestehend aus Blasensäulenreaktor, einem Dünnschichtverdampfer und einer Destillationseinrichtung, die entsprechend der Nummern 1-8 in Fig. 1 verschaltet wurden, durchgeführt. Mit dieser Versuchsanlage war es möglich, die wesentlichen Aspekte der zweistufigen Prozessführung im Labor zu untersuchen. Das zu hydroformylierende Olefin wurde unten, zusammen mit einem Überschuss an Synthesegas und einem, den Katalysator enthaltenden hochsiedenden Lösungsmittel, in die Blasensäule eingebracht. Am Kopf des Reaktors wurde nicht umgesetztes Synthesegas abgetrennt. Die flüssigen Anteile (Restolefin, Aldehyde, Nebenprodukte, hochsiedendes Lösungsmittel, Katalysator) wurden dem Dünnschichtverdampfer zugeleitet, der unter vermindertem Druck betrieben wurde, so dass hier der gebildete Aldehyd zusammen mit den nicht umgesetzten Olefinen von den höhersiedenden Komponenten, in denen der Katalysator gelöst war, getrennt wurde. Als hochsiedendes Lösungsmittel wurde Dioctylphthalat eingesetzt, das mit 20 % Gewichtsanteil im Reaktor vorlag. Die Rhodiumkonzentratiom im Reaktor betrug 100 ppm Rhodium, als Ligand wurde Tris(2.4-ditert.-butylphenyl)phosphit zugesetzt, das P/Rh-Verhältnis betrug 20/1. Die Blasensäule wurde von außen über einen Doppelmantel auf konstant 120°C temperiert, der Betriebsdruck lag bei 50 bar Synthesegas.

Bei den oben angegebenen Reaktionsbedingungen wurde ein Olefinzulauf von 2 kg/h Di-n-buten eingestellt, die Blasensäule hatte ein Volumen von 2,1 Litern. Nachdem sich ein konstantes Umsatzniveau eingestellt hatte, wurden über einen Zeitraum von 100 Stunden die Stoffströme bilanziert. Das über den Dünnschichtverdampfer abgetrennte Gemisch wurde hierfür destillativ in nicht umgesetzte Olefine und die gebildeten Aldehyden aufgetrennt. Aus 200 kg Di-n-buten wurden 156 kg Aldehyde und 77 kg Olefin erhalten, was einem mittleren Umsatz von 61.5 % entspricht. Gleichzeitig wurden 130 g hochsiedende Nebenprodukte gebildet, die sich im Katalysatorkreislauf anreicherten.

Das in der ersten Stufe nicht umgesetzte Olefin wurde in einer zweiten Hydroformylierungsstufe erneut in der Versuchsanlage umgesetzt. Die Reaktionsbedingungen entsprachen denen der ersten Stufe, nur der Zulauf an Olefin wurde auf 1 kg/h reduziert. Als Bilanzzeitraum wurden 77 Stunden gewählt, in dem genau die 77 kg Olefin aus dem Bilanzzeitraum der ersten Stufe umgesetzt wurden. Es wurden 65 kg Aldehyde erhalten. Gleichzeitig wurden 310 g hochsiedende Nebenprodukte gebildet.

Fasst man die Ergebnisse der beiden Bilanzzeiträume zusammen, so wurden in insgesamt 177 Betriebsstunden aus 200 kg Di-n-buten 221 kg Aldehyde erhalten. Dabei wurden 440 g hochsiedende Nebenprodukte gebildet.

### Beispiel 4 (Vergleichsbeispiel, Einstufige Hydroformylierung)

Als Vergleich zu Beispiel 3 wurde unter sonst gleichen Versuchbedingungen 200 kg Di-n-buten innerhalb von 177 Stunden in die Versuchsanlage eingefahren (1.13 kg(Olefin)/h). Dabei wurden insgesamt 198 kg Aldehyd gebildet. Gleichzeitig entstanden 490 g hochsiedende Nebenprodukte.

Der Vergleich der Beispiele 3 und 4 zeigt, dass durch Hydroformylierung des Olefins in zwei Stufen über den gleichen Zeitraum bei der gleichen Menge an eingesetztem Olefin 23 kg mehr Aldehyde erhalten wurden. Daraus resultiert, dass man durch Aufteilung der Hydroformylierungsreaktion in zwei Stufen bessere Raum-Zeit-Ausbeuten erhält als bei einer einstufigen Reaktion. Gleichzeitig zeigt sich, dass in der zweistufigen Fahrweise trotz des über beide Stufen gerechneten höheren Umsatzes insgesamt weniger hochsiedende Nebenprodukte entstehen. Das ist von besonderer Bedeutung, da der Rhodiumkatalysator bei der Aufarbeitung der Oxierungsgemische in den Hochsiedern gelöst bleibt. Je mehr Hochsieder ausgeschleust werden müssen, umso mehr Rhodium muss nachdosiert werden.

### Beispiel 5

### Nonanole durch zweistufige Hydroformylierung von Di-n-buten

### 1. Stufe

In einem 51 Hochdruckautoklaven mit Rührer und elektrischer Heizung wurden 2 000 g Di-n-buten (Zusammensetzung in Tabelle 1, Spalte 2) in Gegenwart eines Kobaltkatalysators bei 175°C und einem Synthesegasdruck von 280 bar 2 Stunden lang hydroformyliert. Der Katalysator wurde hergestellt, indem 640 g einer wässrigen Kobaltacetat-Lösung mit 1 Massen-% Kobalt 7 Stunden bei 170°C und 280 bar mit Synthesegas behandelt wurden. Nach Abkühlen und Entspannen wurden die gebildeten Kobaltcarbonyle durch Extraktion mit den 2 000g Di-n-buten in die organische Phase überführt, die von der wässrigen abgetrennt wurde. Die Konzentration des Katalysators im Di-n-buten betrug 0,020 Massen-% bezogen auf Di-n-buten und gerechnet als Kobaltmetall.

Nach Abkühlen auf 80°C und Entspannung wurde das Hydroformylierungsgemisch durch Behandlung mit 5 gew.-%iger wässriger Essigsäure in Gegenwart von Luft von Kobalt befreit. Das entkobaltete Hydroformylierungsgemisch wurde von der wässrigen Phase abgetrennt.

Das Verfahren wurde unter gleichen Bedingungen viermal durchgeführt. Die entkobalteten Hydroformylierungsgemische wurden vereinigt. Es wurden 9 432 g Hydroformylierungsgemisch erhalten; die Zusammensetzung gemäß GC-Analyse ist in Tabelle 2, Spalte 2 angegeben. Danach betrug der Di-n-buten-Umsatz 67,2 % und die Wertprodukt-Selektivität 93,8 %, entsprechend einer Wertproduktausbeute von 63,1 %. Als Wertprodukte wurden hier und in der Folge Nonanale, Nonanole und deren Formiate betrachtet.

### 2. Stufe

7 500 g entkobaltetes Hydroformylierungsgemisch aus der ersten Stufe wurden zur Rückgewinnung von nicht umgesetzten Olefinen über eine Kolonne destilliert. Die Olefine wurden als Kopffraktion erhalten, der Kolonnensumpf enthielt die Wertprodukte und die Hochsieder. Die Isomerenverteilung in dem rückgewonnenen Octengemisch ist in Tabelle 1, Spalte 3 aufgeführt. Im Vergleich zu frischem Di-n-buten mit 23 Massen-% Dimethylhexenen enthielt das rückgewonnene Olefin mit 45 Massen-% Dimethylhexenen erheblich mehr an diesen reaktionsträgen Olefinen.

2 000 g rückgewonnenes C₈-Kohlenwasserstoffgemisch (91,75 Gew.-% C₈-Olefine, 8,25 Gew.-% C₈-Paraffine) wurden in dem 51 Autoklaven der 1. Stufe bei 185°C und einem Synthesegasdruck von 280 bar 3 Stunden lang hydroformyliert. Der Kobaltkatalysator wurde wie in der 1. Stufe hergestellt und in die Olefinphase überführt, seine Konzentration betrug 0,050 Massen-% bezogen auf das Olefin und gerechnet als Kobaltmetall.

Das Hydroformylierungsgemisch wurde auf 80°C abgekühlt, entspannt und entkobaltet wie in der 1. Stufe beschrieben. Man erhielt 2 448 g entkobaltetes Hydroformylierungsgemisch, dessen Zusammensetzung gemäß GC-Analyse in Tabelle 2, Spalte 3 wiedergegeben ist. Der Olefinumsatz betrug 91 % und die Wertprodukt-Selektivität 83,7 %, entsprechend einer Wertproduktausbeute von 76,2 %.

Der Gesamtolefinumsatz über beide Stufen betrug 97,2 % bei einer Wertprodukt-Selektivität von 90,7 %, entsprechend einer Gesamt-Wertproduktausbeute von 88,2 % bezogen auf eingesetztes Di-n-buten.

### Beispiel 6 (Vergleichsbeispiel, Nonanole aus einstufiger Hydroformylierung von Di-n-buten)

In dem in Beispiel 5 verwendeten 51 Hochdruckautoklaven wurden 2 000 g Di-n-Buten (Zusammensetzung in Tabelle 1, Spalte 2 ) in Gegenwart eines Kobaltkatalysators bei 185°C und einem Synthesegasdruck von 280 bar 3 Stunden lang hydroformyliert. Der Katalysator wurde hergestellt wie in Beispiel 5. Die Konzentration des Katalysators im Di-n-buten betrug 0,040 Massen-%, bezogen auf Di-n-buten und gerechnet als Kobaltmetall.

Nach Abkühlen auf 80°C wurde das Hydroformylierungsgemisch entspannt und durch Behandlung mit 5 gew.-%iger wässriger Essigsäure und Luft von Kobalt befreit. Nach Abtrennung von der wässrigen Phase wurden 2 485 g entkobaltetes Hydroformylierungsgemisch erhalten, dessen mittels GC-Analyse ermittelte Zusammensetzung in Tabelle 2, Spalte 4 aufgeführt ist. Danach wurde ein Di-n-buten-Umsatz von 92 % erzielt, bei einer Wertprodukt-Selektivität von 88,5 %, entsprechend einer Produktausbeute von 81,4 %.

Im erfindungsgemäßen, mehrstufigen Verfahren (Beispiel 5) wurden gegenüber einem einstufigen Verfahren (Beispiel 6) deutlich bessere Umsätze, Selektivitäten und Ausbeuten erzielt.

**Tabelle 1**

| Isomerenverteilung im Einsatzolefin | | |
|---|---|---|
| **Olefine** | **Di-n-buten (Edukt in Bsp. 5, 1. Stufe und Bsp. 6)** **Massen-%** | **Octengemisch (Edukt in Bsp. 5, 2.Stufe)** **Massen-%** |
| Dimethylhexene | 23 | 45 |
| 3-Methylheptene | 62 | 50 |
| n-Octene | 15 | 5 |

**Tabelle 2**

| Zusammensetzung von entkobalteten Hydroformylierungsausträgen (H₂O-frei gerechnet) | | | |
|---|---|---|---|
| | **Bsp. 5, 1. Stufe Massen-%** | **Bsp. 5, 2. Stufe Massen-%** | **Bsp. 6 Massen-%** |
| C₈-Olefine | 27,8 | 6,7 | 6,4 |
| C₈-Paraffine | 2,5 | 10,8 | 3,1 |
| C₉-Aldehyde | 48,8 | 45,2 | 52,7 |
| Nonylformiate | 2,2 | 5,7 | 4,2 |
| C₉-Alkohole | 17,4 | 22,9 | 26,9 |
| Hochsieder | 1,3 | 8,7 | 6,7 |

## Patentansprüche

1. Verfahren zur Herstellung von Alkoholen durch mehrstufige Kobalt- oder Rhodium-katalysierte Hydroformylierung von Olefinen mit 6 bis 24 Kohlenstoffatomen zu Aldehyden,
**dadurch gekennzeichnet,**
**dass** die Olefine
a) in einem Hydroformylierungsschritt bis zu einem Umsatz von 20 bis 98 % hydroformyliert werden,
b) der Katalysator aus dem so erhaltenen flüssigen Reaktoraustrag entfernt wird,
c) das so erhaltene flüssige Hydroformylierungsgemisch in eine Leichtsiederfraktion, enthaltend Olefine und Paraffine und eine Sumpffraktion, enthaltend Aldehyde und/oder Alkohole getrennt wird,
d) die in der Leichtsiederfraktion enthaltenden Olefine in weiteren Verfahrensstufen, umfassend die Verfahrensschritte a, b und c umgesetzt werden
und die Sumpffraktionen der Verfahrensschritte c) aller Verfahrensstufen vereinigt werden, wobei
- jede Verfahrensstufe einen Hydroformylierungsschritt a), einen Katalysatorabtrennungsschritt b) und einen Destillationsschritt c) aufweist, mit der Maßgabe, dass der in b) abgetrennte Katalysator direkt oder nach Aufarbeitung in den Hydroformylierungsschritt a) der jeweiligen Verfahrensstufe zurückgeführt wird, oder
- jede Verfahrensstufe einen Hydroformylierungsschritt a), einen Katalysatorabtrennungsschritt b) aufweist und die vereinigten flüssigen Hydroformylierungsgemische in einem gemeinsamen Destillationsschritt c) in Leichtsieder- und Sumpffraktion getrennt werden, mit der Maßgabe, dass der in den Schritten b) abgetrennte Katalysator direkt oder nach Aufarbeitung in den Hydroformylierungsschritt a) der jeweiligen Verfahrensstufe zurückgeführt wird, oder
- die vereinigten Reaktorausträge aller Hydroformylierungsschritte a) nur einen Katalysatorabtrennungsschritt b) und einen Destillationsschritt c) durchlaufen, mit der Maßgabe, dass der in dem Verfahrensschritt b) abgetrennte Katalysator direkt oder nach Aufarbeitung aufgeteilt und in die Hydroformylierungsschritte a) der einzelnen Verfahrensstufen zurückgeführt wird,
und die vereinigten Sumpffraktionen aus den Destillationsschritten c) hydriert werden oder wobei
- jede Verfahrensstufe einen Hydroformylierungsschritt a) einen Katalysatorabtrennungsschritt b) und bis auf die letzte Verfahrensstufe einen Destillationsschritt c) aufweist, mit der Maßgabe, dass der in b) abgetrennte Katalysator direkt oder nach Aufarbeitung in den Hydroformylierungsschritt a) der jeweiligen Verfahrensstufe zurückgeführt wird und
**dass** die vereinigten Sumpffraktionen der Destillationsschritte c) und der Austrag des Katalysatorabtrennungsschritts b) der letzten Verfahrensstufe hydriert werden.

2. Verfahren nach Anspruch 1
**dadurch gekennzeichnet,**
**dass** die Paraffine aus mindestens einer Leichtsiederfraktion ganz oder teilweise ausgeschleust werden.

3. Verfahren nach einem der Ansprüche 1 oder 2,
**dadurch gekennzeichnet,**
**dass** in jedem Hydroformylierungsschritt a) Kobaltkatalysatoren eingesetzt werden.

4. Verfahren nach einem der Ansprüche 1 oder 2,
**dadurch gekennzeichnet,**
**dass** in jedem Hydroformylierungsschritt a) Rhodiumkatalysatoren eingesetzt werden.

5. Verfahren nach einem der Ansprüche 1 oder 2,
**dadurch gekennzeichnet,**
**dass** in der ersten Verfahrensstufe im Hydroformylierungsschritt a) ein Kobaltkatalysator und in den Hydroformylierungsschritten a) der weiteren Verfahrensstufen ein Rhodiumkatalysator eingesetzt wird.

6. Verfahren nach einem der Ansprüche 1 oder 2,
**dadurch gekennzeichnet**,
das in der ersten Verfahrensstufe im Hydroformylierungsschritt a) ein Rhodiumkatalysator und in den Hydroformylierungsschritten a) der weiteren Verfahrensstufen ein Kobaltkatalysator eingesetzt wird.

7. Verfahren nach einem der Ansprüche 1 bis 6,
**dadurch gekennzeichnet,**
**dass** die flüssigen Reaktorausträge der Hydroformylierungsschritte a) homogene Flüssigphasen sind.

8. Verfahren nach einem der Ansprüche 1 bis 7,
**dadurch gekennzeichnet,**
**dass** der Kobalt- oder Rhodiumkatalysator homogen in den flüssigen Reaktorausträgen der Hydroformylierungsschritte a) gelöst ist.

9. Verfahren nach einem der Ansprüche 1 bis 8,
**dadurch gekennzeichnet,**
**dass** die Olefine in den auf die erste Verfahrensstufe folgenden Hydroformylierungsschritten a) der weiteren Verfahrensstufen jeweils bis zu einem Umsatz von mindestens 50 % hydroformyliert werden.

10. Verfahren nach Anspruch 9,
**dadurch gekennzeichnet,**
**dass** die Olefine in den auf die erste Verfahrensstufe folgenden Hydroformylierungsschritten a) der weiteren Verfahrensstufen jeweils bis zu einem Umsatz von 55 bis 98 % hydroformyliert werden.

11. Verfahren nach einem der Ansprüche 1 bis 10,
**dadurch gekennzeichnet,**
**dass** es zwei Verfahrensstufen umfasst.

## Claims

1. A process for preparing alcohols by multistage cobalt- or rhodium-catalysed hydroformylation of olefins having 6 to 24 carbon atoms to give aldehydes, **characterized by** the steps of ,
a) hydroformylating the olefins in a hydroformylation step to a conversion of from 20 to 98%,
b) removing the catalyst from the resulting liquid reactor discharge,
c) separating the resulting liquid hydroformylation mixture into a low-boiler fraction comprising olefins and paraffins and a bottoms fraction comprising aldehydes and/or alcohols,
d) reacting the olefins present in the low-boiler fraction in further process stages comprising the process steps a, b and c
and combining the bottoms fractions of process steps c) of all process stages
where
- each process stage has a hydroformylation step a), a catalyst removal step b) and a distillation step c), with the proviso that the catalyst separated off in b) is returned, directly or after work-up, to the hydroformylation step a) of the respective process stage
or
- each process step has a hydroformylation step a), a catalyst removal step b), and the combined liquid hydroformylation mixtures are separated in a common distillation step c) into a low-boiler fraction and bottoms fraction, with the proviso that the catalyst separated off in steps b) is returned, directly or after work-up, to the hydroformylation step a) of the respective process stage
or
- the combined reactor discharges of all hydroformylation steps a) pass through only one catalyst removal step b) and one distillation step c), with the proviso that the catalyst separated off in process step b) is divided, directly or after work-up, and returned to the hydroformylation steps a) of the individual process stages and the combined bottoms fractions from the distillation steps c) are hydrogenated,
or where
- each process stage has a hydroformylation step a), a catalyst removal step b) and, apart from the last process stage, a distillation step c), with the proviso that the catalyst separated off in b) is returned, directly or after work-up, to the hydroformylation step a) of the respective process stage and in that the combined bottoms fractions of distillation steps c) and the discharge of catalyst removal step b) of the last process stage are hydrogenated.

2. A process according to claim 1,
**characterized in that**
the paraffins are partially or completely removed from at least one low-boiler fraction.

3. A process according to either of claims 1 and 2,
**characterized in that**
in each hydroformylation step a) cobalt catalysts are used.

4. A process according to either of claims 1 and 2,
**characterized in that**
in each hydroformylation step a) rhodium catalysts are used.

5. A process according to either of claims 1 and 2,
**characterized in that**
in the first process stage in the hydroformylation step a) a cobalt catalyst is used and in the hydroformylation steps a) of the further process stages a rhodium catalyst is used.

6. A process according to either of claims 1 and 2,
**characterized in that**
in the first process stage in the hydroformylation step a) a rhodium catalyst is used and in the hydroformylation steps a) of the further process stages a cobalt catalyst is used.

7. A process according to any of claims 1 to 6,
**characterized in that**
the liquid reactor discharges of hydroformylation steps a) are homogeneous liquid phases.

8. A process according to any of claims 1 to 7,
**characterized in that**
the cobalt or rhodium catalyst is dissolved homogeneously in the liquid reactor discharges of hydroformylation steps a).

9. A process according to any of claims 1 to 8,
**characterized in that**
the olefins in the hydroformylation steps a) of the further process stages which follow the first process stage are in each case hydroformylated to a conversion of at least 50%.

10. A process according to claim 9,
**characterized in that**
the olefins in the hydroformylation steps a) of the further process stages which follow the first process stage are in each case hydroformylated to a conversion of from 55 to 98%.

11. A process according to any of claims 1 to 10,
**characterized in that** comprises two process stages.

## Revendications

1. Procédé de fabrication d'alcools par hydroformylation, en plusieurs étapes, catalysée par du cobalt ou du rhodium, d'oléfines portant 6 à 24 atomes de carbone en aldéhydes,
**caractérisé en ce que**
a) les oléfines sont hydroformylées dans une opération d'hydroformylation jusqu'à une conversion de 20 à 98 %,
b) on élimine le catalyseur de la sortie de réacteur liquide ainsi obtenue,
c) on sépare le mélange d'hydroformylation liquide ainsi obtenu en une fraction à bas point d'ébullition, contenant des oléfines et des paraffines, et une fraction de fond, contenant des aldéhydes et/ou des alcools,
d) on convertit les oléfines contenues dans la fraction à bas poids moléculaire dans d'autres étapes du procédé, comprenant les opérations a, b et c,
on réunit les fractions de fond des opérations c) de toutes les étapes du procédé, et
- chaque étape présente une opération d'hydroformylation a), une opération de séparation du catalyseur b) et une opération de distillation c), étant précisé que le catalyseur séparé dans l'opération b) est reconduit à l'étape concernée directement ou après régénération au cours de l'opération d'hydroformylation a),
ou bien
- chaque étape comporte une opération d'hydroformylation a), une opération de séparation du catalyseur b) et les mélanges d'hydroformylation liquides réunis sont séparés dans une opération de distillation c) commune en une fraction à bas point d'ébullition et de fond, étant précisé que le catalyseur séparé dans les opérations c) est reconduit à l'étape de procédé concernée directement ou après régénération au cours de l'opération d'hydroformylation a),
ou bien
- les extraits de réacteur réunis de toutes les opérations d'hydroformylation a) passent par une seule opération de séparation du catalyseur b) et une seule opération de distillation c), étant précisé que le catalyseur séparé au cours de l'opération b) est divisé directement ou après régénération et reconduit aux différentes étapes de procédé au cours des opérations d'hydroformylation a),
et les fractions de fond réunies venant des opérations de distillation c) sont hydratées,
ou bien
- chaque étape d'une opération d'hydroformylation a) comporte une opération de séparation du catalyseur b) et, à part la dernière étape de procédé, une opération de distillation c), étant précisé que le catalyseur séparé au cours de b) est reconduit à l'étape de procédé concernée directement ou après régénération au cours de l'opération d'hydroformylation a) et
les fractions de fond réunies des opérations de distillation c) et la sortie de l'opération de séparation du catalyseur b) de la dernière étape de procédé sont hydratées.

2. Procédé selon la revendication 1
**caractérisé en ce que**
les paraffines sont éclusées complètement ou partiellement d'au moins une fraction à bas point d'ébullition.

3. Procédé selon l'une quelconque des revendications 1 ou 2,
**caractérisé en ce que**
on met en oeuvre des catalyseurs au cobalt dans chaque opération d'hydroformylation a).

4. Procédé selon l'une quelconque des revendications 1 ou 2,
**caractérisé en ce que**
on met en oeuvre des catalyseurs au rhodium dans chaque opération d'hydroformylation a).

5. Procédé selon l'une quelconque des revendications 1 ou 2,
**caractérisé en ce que**
on met en oeuvre, lors de la première étape dans l'opération d'hydroformylation a), un catalyseur au cobalt et, dans les opérations d'hydroformylation a) des autres étapes, un catalyseur au rhodium.

6. Procédé selon l'une quelconque des revendications 1 ou 2,
**caractérisé en ce que**
on met en oeuvre, lors de la première étape dans l'opération d'hydroformylation a), un catalyseur au rhodium et, dans les opérations d'hydroformylation a) des autres étapes, un catalyseur au cobalt.

7. Procédé selon l'une quelconque des revendications 1 ou 6,
**caractérisé en ce que**
les sorties de réacteur liquides des opérations d'hydroformylation a) sont des phases liquides homogènes.

8. Procédé selon l'une quelconque des revendications 1 ou 7,
**caractérisé en ce que**
on dissout le catalyseur au cobalt ou au rhodium de façon homogène dans les sorties de réacteur liquides des opérations d'hydroformylation a).

9. Procédé selon l'une quelconque des revendications 1 ou 8,
**caractérisé en ce que**
on hydroformyle les oléfines dans les opérations d'hydroformylation a), suivant la première étape de procédé, des autres étapes de procédé jusqu'à une conversion d'au moins 50 %.

10. Procédé selon la revendication 9,
**caractérisé en ce que**
on hydroformyle les oléfines dans les opérations d'hydroformylation a), suivant la première étape de procédé, des autres étapes de procédé jusqu'à une conversion d'au moins 55 à 98 %.

11. Procédé selon l'une quelconque des revendications 1 à 10,
**caractérisé en ce que**
il comporte deux étapes de procédé.
